(19) Europäisches Patentamt
European Patent Office
Office européen des brevets

(11) Veröffentlichungsnummer: **0 244 833 B1**

(12) # EUROPÄISCHE PATENTSCHRIFT

(45) Veröffentlichungstag der Patentschrift: **24.06.92**

(21) Anmeldenummer: **87106522.3**

(22) Anmeldetag: **06.05.87**

(51) Int. Cl.⁵: **C07D 309/30**, C07C 323/50, C07C 229/36, C07C 59/68, C07D 309/32, C07D 409/12, C07D 407/10, C07D 405/10, C07D 409/10, A61K 31/365

(54) **4(R)-substituierte 6(S)-Phenoxymethyl-, 6(S)-beta-Phenyläthyl- und 6(S)-beta-Styryl-tetrahydropyran-2-one, ein hochstereoselektives Verfahren zu ihrer Herstellung, pharmazeutische Präparate auf Basis dieser Verbindungen und ihre Verwendung.**

(30) Priorität: **09.05.86 DE 3615620**

(43) Veröffentlichungstag der Anmeldung: **11.11.87 Patentblatt 87/46**

(45) Bekanntmachung des Hinweises auf die Patenterteilung: **24.06.92 Patentblatt 92/26**

(84) Benannte Vertragsstaaten: **AT BE CH DE ES FR GB GR IT LI LU NL SE**

(56) Entgegenhaltungen:
**EP-A- 0 024 348**
**DE-A- 2 134 094**
**DE-A- 2 822 848**

**TETRAHEDRON LETTERS, Band 23, Nr. 42, Seiten 4305-4308, Oktober 1982, London, GB; Y. YANG et al.: "Mevinic acids and analogues: Preparation of a key chiral intermediate"**

(73) Patentinhaber: **HOECHST AKTIENGESELLSCHAFT**
**Postfach 80 03 20**
**W-6230 Frankfurt am Main 80(DE)**

(72) Erfinder: **Jendralla, Heiner, Dr.**
**Unter den Nussbäumen 5**
**W-6233 Kelkheim (Taunus)(DE)**
Erfinder: **Beck, Gerhard, Dr.**
**Gustav-Freytag-Strasse 24**
**W-6000 Frankfurt an Main 1(DE)**
Erfinder: **Wess, Günther, Dr.**
**Hainstrasse 35**
**W-6455 Erlensee(DE)**
Erfinder: **Kerekjarto, Bela, Dr.**
**Weilbächer Wälder FA8**
**W-6238 Hofheim am Taunus(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

**PATENT ABSTRACTS OF JAPAN**, Band 6, Nr. 68 (C-100) (946), 30. April 1982; & JP - A - 577480 (SANKYO) 14.01.1982

**PATENT ABSTRACTS OF JAPAN**, Band 5, Nr. 54 (C-50) (726), 15. April 1981; & JP - A - 565474 (SANKY0) 10.01.1981

**TETRAHEDRON LETTERS**, Band 27, Nr. 36, Seiten 4709-4712, September 1986, London, GB; W. Bartmann et al.: "Convenient two-step stereospecific hydroxy-substitution with retention in beta-hydroxy-substitution with retention in beta-hydroxy-delta-lactones"

## Beschreibung

Es ist bekannt, daß das Enzym 3-Hydroxy-3-methylglutaryl-Coenzym-A-Reduktase (HMG-CoA-Reduktase) die Bildung von Mevalonsäure aus 3-Hydroxy-3-methylglutaryl-Coenzym A (HMG-CoA) katalysiert. Diese Reaktion spielt eine zentrale Rolle bei der Biosynthese des Cholesterins. Derivate der 3-Hydroxy-3-methyl-gutarsäure (HMG) und der Mevalonsäure sind als Hemmer der Cholesterinbiosynthese mehrfach beschrieben worden.

So beschreiben G.E. Stokker et al (J. Med. Chem. 28, 347 - 358 (1985)) in 5-Stellung substitutiertes 3,5-Dihydroxypentansäurederivate und deren 4-Hydroxy-Lactone und W.F. Hoffmann et al. (J. Med. Chem. 29, 159 ff. (1986))) in 7-Stellung durch Aryl substituierte 3,5-Dihydroxy-6-heptansäurederivate und deren 4-Hydroxy-Lactone, welche die HMG-CoA-Reduktase hemmen.

Es wurde bereits vorgeschlagen (vgl. EP-A-0 216 127, Stand der Technik nach Artikel 54 (3) EPü) und EP-A-0 217 092, Stand der Technik nach Artikel 54 (3) EPü, daß 6-Phenoxymethyl-4-hydroxytetrahydropyran-2-one und 6(S)-$\beta$-Phenylethyl und 6(S)-$\beta$-Styryltetrahydropyran-2-one der darin angegebenen allgemeinen Formeln sowie die entsprechenden Dihydroxycarbonsäuren, deren Salze und Ester Hemmstoffe der HMG-CoA-Reduktase sind und daher als Arzneimittel, insbesondere zur Prophylaxe und Therapie der Hypercholesterinämie, verwendet werden können.

Die in den genannten Publikationen und Anmeldungen beschriebenen Verbindungen, die die HMG-CoA-Reduktase hemmen, weisen die unveränderte 4(R)-Hydroxygruppe bei den Lactonen bzw. 3(R)-Hydroxygruppe bei den Dihydroxycarbonsäuren auf.

Die wenigen Beispiele für eine Veränderung oder einen Ersatz dieser Hydroxygruppen: 4(S)-Konfiguration (G.E. Stokker et al., J. Med. Chem. 28, 347 (1985)), Enolgruppierung oder Methylgruppe (G.E. Stokker et al., J. Med. Chem. 28, 347 (1985)), Acetylierung (A. Endo. J. Med. Chem. 28, 401 (1985)) bewirkten entspechend den Literaturangaben nur äußerst geringe oder gar keine Inhibierung der HMG-CoA-Reduktase.

Es wurde nun gefunden, daß Verbindungen entsprechend den deutschen Patentanmeldung P 35 30 798.6 bzw. P 35 43 336.1 (= US-Patentanmeldung Nr. 900 848) sowie P 35 30 497.8 (= US-Patentanmeldung Nr. 900 887), die statt der 4(R)- bzw. 3(R)-Hydroxygruppe den Rest $R^7$-A- tragen, ebenfalls die HMG-CoA-Reduktase hemmen und daher als Arzneimittel, insbesondere zur Prophylaxe und Therapie der Hypercholesterinämie, verwendet werden können.

Die Erfindung betrifft daher Tetrahydropyranone der allgemeinen Formel V

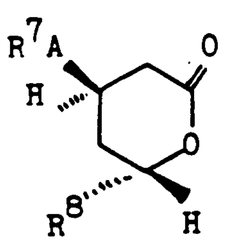

V

worin bedeuten:

A -S-, -NH-, $NR^7$ oder $-CHR^7-$

$R^7$

a) einen geradkettigen oder verzweigten Alkylrest mit 1 - 3 Kohlenstoffatomen, der substituiert sein kann

aa) mit einer Hydroxygruppe

ab) mit einer Amino- oder Ammonium-Gruppe

ac) mit einer Carboxylgruppe $CO_2H$, deren Methyl-, Ethyl- oder Benzylester, Methyl- oder Dimethylamid, Natrium-, Kalium- oder Ammonium-Salz

ad) mit einem Phenylrest

ae) mit 1 bis 3 Halogenatomen

b) einen Alkanoylgruppe

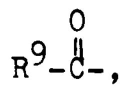

worin $R^9$ ein geradkettiger oder verzweigter Alkylrest mit 1 bis 4 C-Atomen ist, der substituiert sein kann mit den unter aa) bis ae) angegebenen Gruppen

c) eine Carboxylgruppe, deren Methyl-, Ethyl- oder Benzylester, Methyl- oder Dimethylamid, Natrium-, Kalium- oder Ammonium-Salz

d) Wasserstoff

$R^8$ die Strukturelemente der Formeln VI oder VII

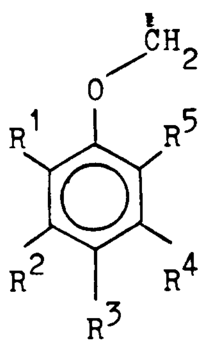

oder

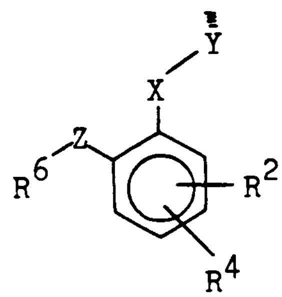

VI VII

worin bedeuten

X-Y einen Rest der Formel trans -CH = CH- oder $-CH_2-CH_2-$

Z eine $-CH_2-$ oder $-CH_2-CH_2-$Gruppe

$R^1$, $R^5$ gleich oder verschieden sind und

a) Wasserstoff oder Halogen

b) Cycloalkyl mit 4 bis 8 C-Atomen oder einen Phenylrest, der im Kern 1- bis 3-fach substituiert sein kann mit Halogen, Trifluormethyl und/oder Alkyl oder Alkoxy mit je 1 bis 4 C-Atomen oder

c) einen geradkettigen oder verzweigten Alkylrest mit 1 bis 18 Kohlenstoffatomen oder einen geradkettigen oder verzweigten Alkenylrest mit 2 bis 18 Kohlen stoffatomen bedeuten, wobei die Alkyl- bzw. Alkenylreste ihrerseits 1 bis 3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettig oder verzweigten Alkenyloxy- oder Alkinyloxyresten mit 3 bis 6 Kohlenstoffatomen,

β) Halogen, Hydroxy, Cycloalkyl mit 3 bis 7 C-Atomen, unsubstituierten Phenyl, α- oder β-Thienylresten, oder Phenyl-, α- oder β-Thienylresten, welche ihrerseits im Kern 1- bis 3-fach substituiert sind mit Halogen, Trifluormethyl und/oder Alkyl oder Alkoxy mit 1 bis 4 C-Atomen,

γ) unsubstituierten Phenoxy-, Benzyloxy-, α- oder β-Thienyloxyresten, oder Phenoxy-, Benzyloxy-, α- oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen.

δ) der Gruppe

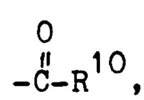

wobei $R^{10}$ bedeutet:

einen geradkettigen oder verzweigten Alkyl- oder Alkenylrest mit bis zu 8 Kohlenstoffatomen, oder einen Cycloalkyl oder Cycloalkenylrest mit jeweils 3 bis 8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1 bis 4 C-Atomen, oder einen Pyridylrest,

$R^2$ und $R^4$, gleich oder verschieden sind und Wasserstoff, Alkyl mit 1 bis 4 C-Atomen, Halogen oder Alkoxy mit 1 bis 4 C-Atomen bedeuten, und

$R^3$ Wasserstoff, Alkyl oder Alkenyl mit bis zu 4 C-Atomen Halogen oder Alkoxy mit 1 bis 4 C-Atomen ist.

$R^6$ einen cycloaliphatischen Kohlenwasserstoffrest mit 3 bis 7 Kohlenstoffatomen, einen Phenylrest, der im Kern 1- bis 3-fach substituiert sein kann mit Halogen, Trifluormethyl, Alkyl oder Alkoxy mit je 1 bis 6 C-Atomen oder mit Hydroxymethyl, oder einen Furyl-, Thienyl- oder Pyridylrest, wobei die heteroaromatischen Reste 1- bis 2-fach substituiert sein können mit Halogen, Trifluormethyl, Alkyl oder Alkoxy mit je 1 bis 6 C-Atomen, sowie die entsprechenden offenkettigen Hydroxycarbonsäuren der Formel V′

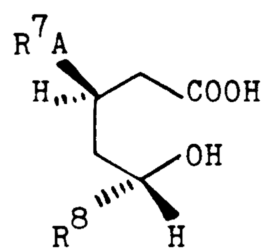

V'

worin A, $R^7$ und $R^8$ die zu Formel V angegebenen Bedeutungen haben, deren phamakologisch verträgliche Salze mit Basen und deren pharmakologisch verträgliche Ester.

Bevorzugter Gegenstand der Erfindung sind Verbindungen der allgemeinen Formel V bzw. V′ worin bedeuten:

A -S-

$R^7$

a) einen geradkettigen oder verzweigten Alkylrest mit 1 bis 2 Kohlenstoffatomen, der substituiert sein kann mit

aa) einer Hydroxygruppe

ab) einer Amino- oder Ammonium-Gruppe

ac) einer Carboxylgruppe, deren Methyl-, Ethyl- oder Benzylester, Methyl- oder Dimethylamid, Natrium-, Kalium- oder Ammoniumsalz

ad) einen Phenylrest

ae) einem Fluor- oder Chloratom

b) eine Alkanoylgruppe

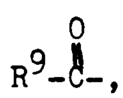

worin $R^9$ ein Methyl- oder Ethylrest ist, der substituiert sein kann mit einer Hydroxygruppe oder 1 bis 3 Fluoratomen

c) eine Carboxylgruppe, deren Methyl- oder Ethylester, Methyl- oder Dimethylamid, Natrium-, Kalium- oder Amminiumsalz,

d) Wasserstoff

$R^8$ die Strukturelemente der Formeln VI oder VII

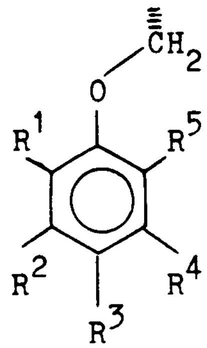

oder

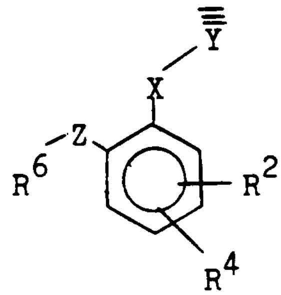

VI VII

worin

X-Y einen Rest der Formel trans -CH = CH- und

Z eine $CH_2$-Gruppe darstellen,

$R^1$, $R^5$ gleich oder verschieden sind und

a) Wasserstoff oder Chlor,

b) einen geradkettigen oder verzweigten Alkylrest mit 1 bis 6 Kohlenstoffatomen, wobei der Alkylrest seinerseits 1- bis 2-fach substituiert sein kann mit Phenyl- oder Phenoxy-Resten, die ihrerseits im Kern substituiert sein können mit Fluor oder Chlor, darstellen.

$R^2$, $R^4$ gleich oder verschieden sind und Wasserstoff, Methyl, Ethyl, Fluor oder Chlor sind,

$R^3$ Wasserstoff, Methyl oder Chlor,

$R^6$ einen cycloaliphatischen Kohlenwasserstoffrest,mit 5 bis 6 C-Atomen, einen Phenylrest, der im Kern substituiert sein kann mit Fluor, Chlor, Trifluormethyl, Methyl, Ethyl, Methoxy, Ethoxy, darstellen.

Besonders bevorzugter Gegenstand der Erfindung sind Verbindungen der allgemeinen Formel V bzw. V′, worin bedeuten

A -S-

$R^7$

a) einen Alkylrest mit 1 oder 2 Kohlenstoffatomen, der substituiert sein kann mit

aa) einer Hydroxygruppe

ab) einer Amino- oder Ammonium-Gruppe

ac) einer Carboxylgruppe, deren Methylester, Natrium-, Kalium- oder Ammonium-Salze

ad) einem Phenylrest

ae) einem Fluoratom

b) einer Acetyl- oder Trifluoracetylgruppe

$R^8$ die Strukturelemente der Formeln VI oder VII

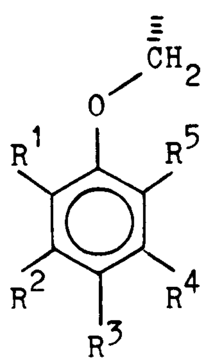

oder

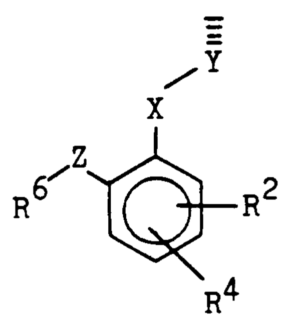

VI VII

worin

X-Y ein Rest der Formel trans -CH = CH- und

Z eine $-CH_2$-Gruppe sind,

$R^1$, $R^5$ gleich oder verschieden sind und
a) Chlor,
b) geradkettiger oder verzweigter Alkylrest mit 1 bis 3 C-Atomen, wobei der Alkylrest seinerseits 1 bis 2-fach substituiert sein kann mit Phenyl- oder Phenoxyresten, die ihrerseits im Kern substituiert sein können mit Fluor, und

$R^2$, $R^4$ Wasserstoff sind

$R^3$ Chlor oder Methyl und

$R^6$ Cyclohexyl-, unsubstiutierter Phenylrest oder Phenylrest substituiert mit Chlor oder Fluor sind.

Die Erfindung betrifft ferner ein Verfahren zur Herstellung von Verbindungen der Formel V bzzw. V' sowie von deren pharmakologisch verträglichen Salzen mit Basen und deren pharmakologisch verträglichen Estern und die Verwendung von entsprechenden Verbindungen zur Herstellung von Arzneimitteln für die Prophylaxe und Therapie der Arteriosklerose und Hypercholesterinämie und pharmazeutische Präparate, die entsprechende Verbindungen enthalten.

Das Verfahren zur Herstellung der Verbindungen der Formel V bzw. V' ist dadurch gekennzeichnet, daß man

a) Verbindungen der allgemeinen Formeln I oder II

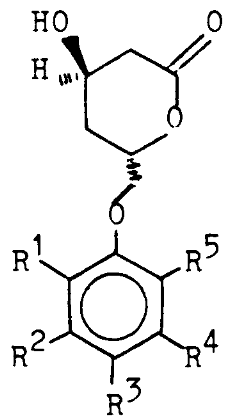

I

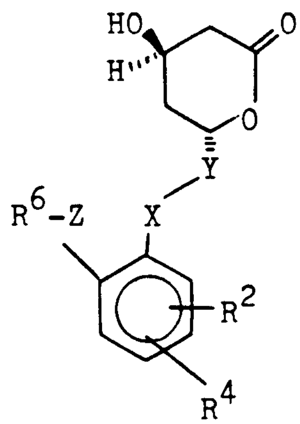

II

worin $R^1$, $R^2$, $R^3$, $R^4$, $R^5$, $R^6$, X, Y, Z die zur Formel V angegebene Bedeutung haben, mit einem wasserabspaltenden Reagenz z.B. mit dem Burgess-Reagenz (E.M. Burgess et al., J. Org. Chem. 38, 26 (1973)) zu den $\alpha,\beta$-ungesättigten Laktonen der allgemeinen Formeln III oder IV umsetzt

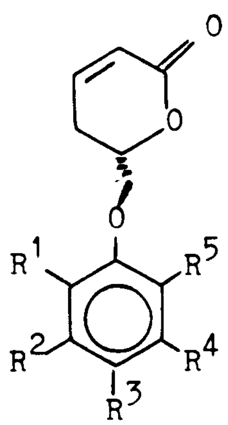

III

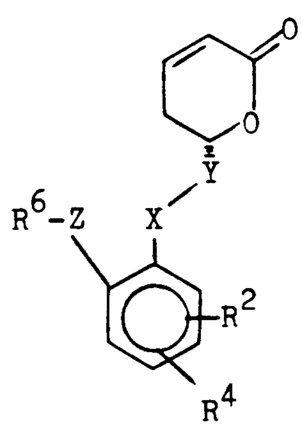

IV

oder die Vorstufen der Formeln I' und II'

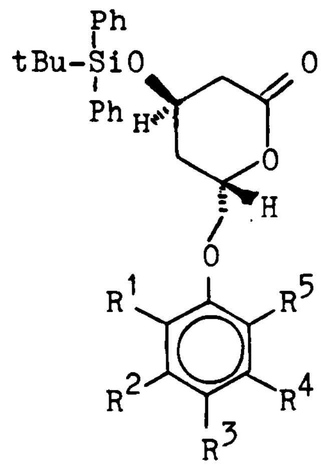

I'

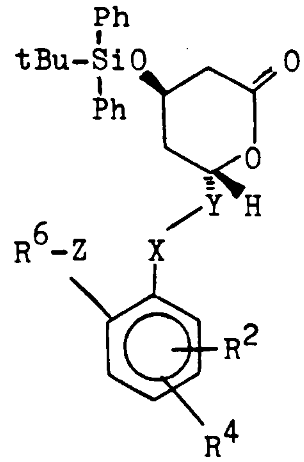

II'

worin $R^1$ bis $R^6$, X, Y und Z die zu Formel V angegebenen Bedeutungen haben, durch Desiloxylierung in die $\alpha,\beta$-ungesättigten Lactone der Formeln III oder IV umsetzt und

b) die $\alpha,\beta$-ungesättigten Lactone der Formeln III oder IV mit nucleophilen Verbindungen $R^7AH$, wie z.B. $R^7$-substituierten Mercaptanen, Thiolsäuren, Aminen,

C-H-aciden Verbindungen, zu Verbindungen der Formel V, worin $R^7$ und A die zu Formel V angegebenen Bedeutungen haben und $R^8$ das Strukturelement VI, worin $R^1$ bis $R^5$ die angegebenen Bedeutungen haben, oder das Strukturelement VII, worin $R^2$, $R^4$ und $R^6$ die angegebenen Bedeutungen haben und X-Y die trans CH = CH-Gruppe darstellt, bedeutet, umsetzt und

gegebenenfalls eine erhaltene Verbindung V, worin X-Y die trans CH = CH-Gruppe darstellt, hydriert zu einer Verbindung V, worin X-Y die $CH_2$-$CH_2$-Gruppe darstellt,

gegebenenfalls eine erhaltene Verbindung der Formel V in die offenkettige Hydroxycarbonsäure der Formel V' oder deren Ester oder Salz überführt, oder

gegebenenfalls ein Salz oder einen Ester in die freie Hydroxycarbonsäure überführt.

Bei dem Verfahren können eine oder mehrere der Gegebenenfallsmaßnahmen in beliebiger Reihenfolge durchgeführt werden.

Bei der Umsetzung mit nucleophilen Verbindungen $R^7AH$ entstehen unter basischer Katalyse stereospezifisch die 4(R)-Michael-Additionsprodukte (vgl. Schema 1, V). Die 4(S)-Isomeren von V wurden bei diesen Additionen nur in sehr geringen Mengen (< 1 bis 2 %) oder gar nicht gebildet.

Das Verfahren zur Herstellung von Verbindungen V mit $R^8$ = Strukturelement VI (Schema 1, Verbindung Va) bzw. mit $R^8$ = Strukturelement VII mit X-Y = trans CH = CH (Schema 1, Verbindung Vb) ist in dem folgenden Schema 1 anhand eines Beispiels erläutert:

Schema 1:

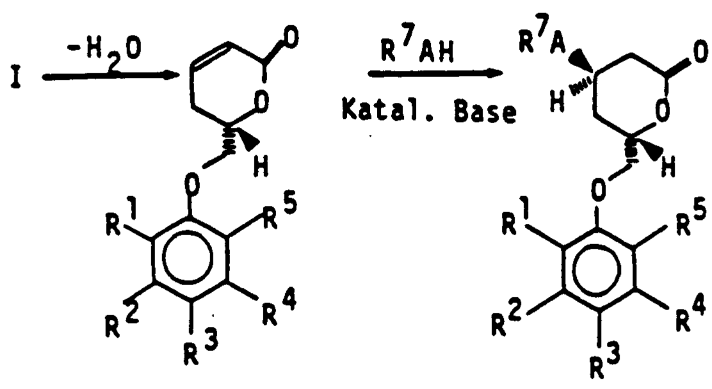


III V

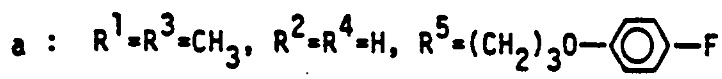

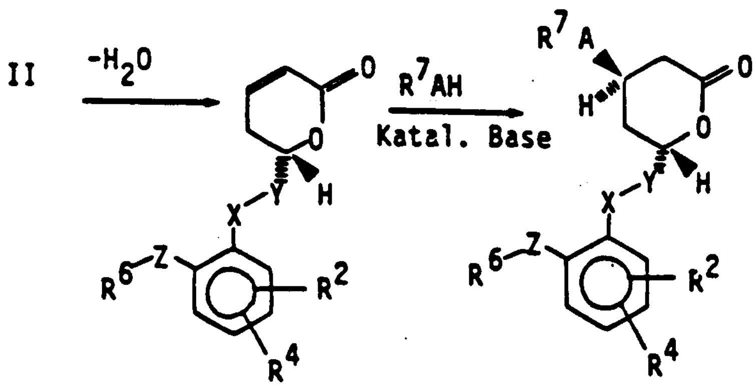


IV V

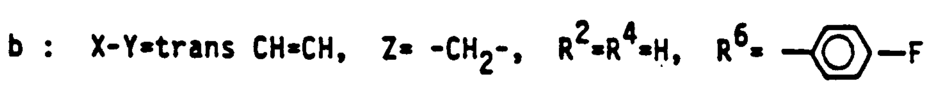

Die Herstellung der optisch reinen Ausgangsverbindungen der Formeln I und II wird in EP-A-0 216 127 und EP-A-0 217 092 beschrieben.

In dem nachfolgenden Schema 2 ist die Synthese von Verbindungen der Formel I bzw. I′ sowie die Überführung in die $\alpha,\beta$-ungestättigten Lactone III anhand eines Beispiels erläutert.

Schema 2: Synthese von III a

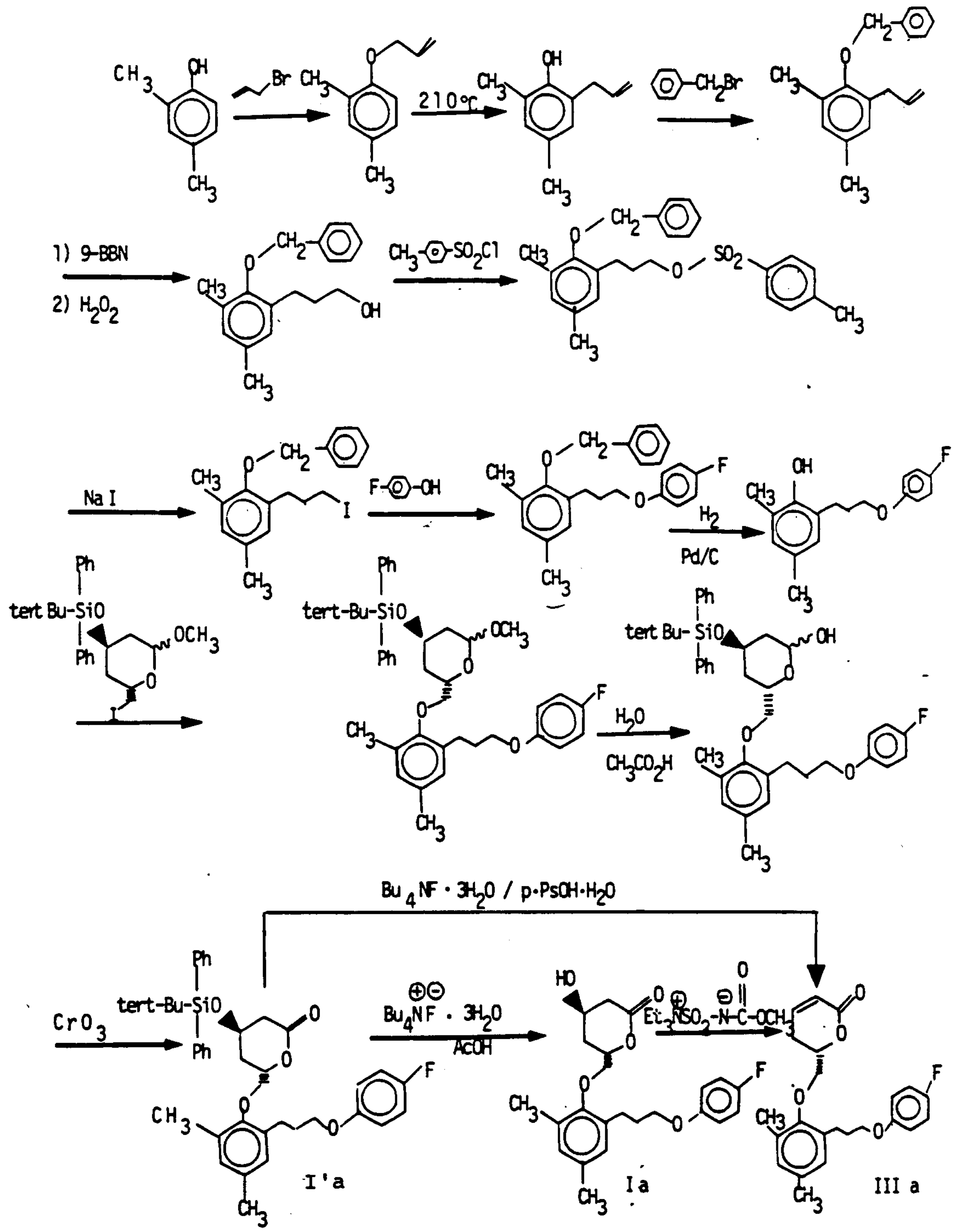


In dem nachfolgenden Schema 3 ist die Herstellung der Ausgangsverbindungen II sowie die Überführung in die α,β-ungesättigten Lactone IV anhand eines Beispiels erläutert:

Schema 3: Synthese von IV b

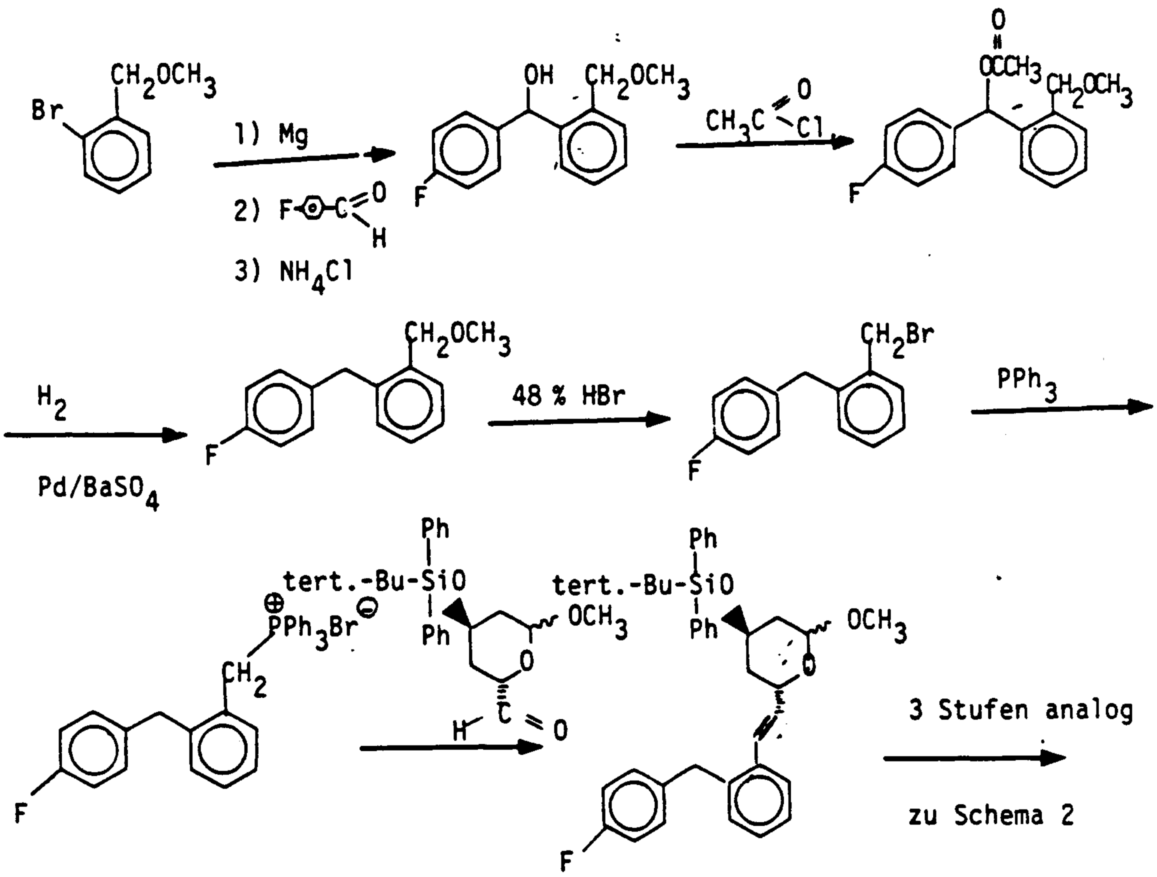


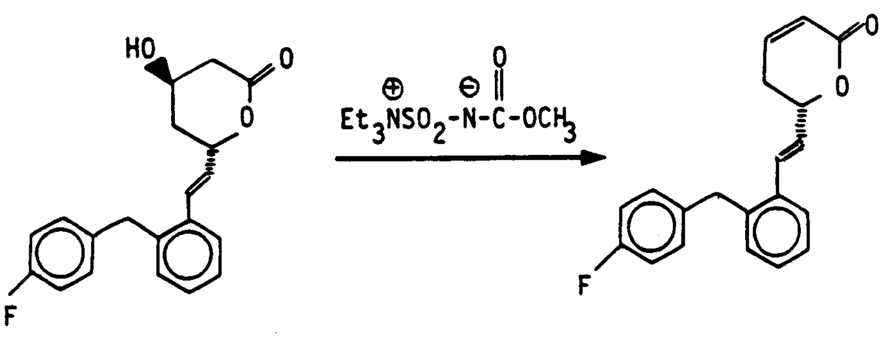


II b

IV b

Die Wasserabspaltung aus Verbindungen der Formel I wobei Verbindungen der Formel III entstehen, wie z.B. aus der Verbindung Ia gemäß Schema 2 zu Verbindung IIIa wird zweckmäßig durch Erwärmen mit Burgess-Reagenz (E.M. Burgess et al., J. Org. Chem. 38, 26 (1973)) vorgenommen, jedoch kommen auch andere gängige Methoden wie Phosphoroxychlorid/Base, Tosylchlorid oder Mesylchlorid/Base, p-Tolylchlorothioformiat (W.H. Rastetter et al., J. Org. Chem. 45, 3149 (1980)), Sulfuran-Reagenzien (J.C. Martin et al., JACS 93, 4327 (1971), JACS 94, 5003 (1972)), Triphenylphosphin/Tetrachlormethan (R. Appel et al., Chem. Ber. 109, 3446 (1976)), Methyltriphenoxyphosphoniumjodid (C.W. Spangler et al., J. Chem. Soc. Perkin I, 2287 (1981)), Erwärmen in Benzol oder Toluol unter saurer Katalyse, Erwärmen in DMSO oder HMPT (R.S. Monson et al., J. Org. Chem. 36, 3827 (1971)), Erwärmen mit Aluminiumoxid (D. Dautzenberg et al., J. Org. Chem. 45 3149 (1980)) in Betracht.

Die Abspaltung von tert.-Butyl-diphenylsilanol aus I'a zu IIIa (Schema 2) erfolgt; z.B. mit Tetrabutylammoniumfluorid/para-Toluolsulfonsäure bereits bei Raumtemperatur.

Die Wasserabspaltung aus Verbindungen II, wobei Lactone IV entstehen, wie z.B. aus IIb zu IVb (Schema 3) wird zweckmäßig durch Erwärmen mit Burgess-Reagenz (E.M. Burgess et al., J. Org. Chem. 38, 26 (1973)) vorgenommen, jedoch kommen auch die obengenannten alternativen Methoden in Betracht. Zur Herstellung des Lactone IV ist dieses Verfahren bevorzugt.

Die Umsetzung der $\alpha,\beta$-ungesättigten Lactone II bzw. IV mit nucleophilen Verbindungen $R^7AH$ erfolgt in Gegenwart basischer Katalysatoren wie z.B. Triethylamin, Natriumhydrid, Natriumcarbonat oder Natriumhydroxid. Die Reaktion wird in An- oder Abwesenheit eines Lösungsmittels sie z.B. Ethanol oder Benzol, vorzugsweise bei Raumtemperatur durchgeführt.

Die Ringöffnung zu Verbindungen V' erfolgt z.B. im alkalischen Medium in Gegenwart von Natriumhydroxid.

Während bei der Addition von z.B. Thioessigsäure, Benzylmercaptan, $\beta$-Mercaptoethanol oder L-Cystein stereospezifisch die 4(R)-Additionsprodukte V entstehen, kann bei der Addition von einem Äquivalent Amin, wie z.B. Benzylamin neben der gewünschten Additionsreaktion zu Verbindungen V zusätzlich eine Öffnung des Lacton-Rings zum Amid stattfinden. In solchen Fällen ist es vorteilhaft, mit einem Aminüberschuß zu arbeiten und die Doppeladdukte herzustellen, und das Amid zur offenkettigen Hydroxycarbonsäure V' zu verseifen.

Die $\alpha,\beta$-ungesättigten Lactone der Formeln III bis VI sind neu. Die Erfindung betrifft daher auch die Verbindungen der Formeln III und IV sowie Verfahren zu deren Herstellung.

Das Enzym HMG-CoA-Reduktase ist in der Natur weit verbreitet. Es katalysiert die Bildung von Mevalonsäure aus HMG-CoA. Diese Reaktion ist ein zentraler Schritt der Cholesterin-Biosynthese (I.R. Sabine, 3-Hydroxy-3-methylglutaryl Coenzym A Reduktase, CRC Press, 1983). Hohe Cholesterinspiegel werden mit einer Reihe von Erkrankungen wie z.B. koronarer Herzkrankheit oder Atherosklerose in Verbindungen gebracht. Daher ist die Senkung erhöhter Cholesterinspiegel zur Vorbeugung und Behandlung solcher Erkrankungen ein therapeutisches Ziel. Ein Ansatzpunkt liegt in der Hemmung bzw. Verminderung der endogenen Cholesterinsynthese. Hemmstoffe der HMG-CoA-Reduktase blockieren die Cholesterin-Biosynthese auf einer frühen Stufe. Sie eignen sich daher zur Vorbeugung und Behandlung von Erkankungen, die durch einen erhöhten Cholesterinspiegel verursacht werden. Eine Verminderung der endogenen Synthese führt zu einer erhöhten Aufnahme von Cholesterin aus dem Plasma in die Zellen. Ein zusätzlicher Effekt läßt sich durch gleichzeitige Gabe Gallensäuren-bindender Stoffe wie Anionenaustauscher erzielen. Die erhöhte Gallensäurenausscheidung führt zu einer verstärkten Neusynthese und damit zu einem erhöhten Chlolesterinabbau (M.S. Brown, P.T. Kovanen, J.L. Goldstein, Science 212, 628 (1981); M.S. Brown, J.L. Goldstein, Spektrum der Wissenschaft 1985, 96). Die erfindungsgemäßen Verbindungen sind Hemmstoffe der HMG-CoA-Reduktase. Sie eignen sich daher zur Hemmung bzw. Verminderung der Cholesterin-Biosynthese und damit zu Vorbeugung oder Behandlung von Erkrankungen, die durch erhöhte Cholesterinspiegel verurusacht werden, insbesondere koronare Herzkrankheit, Atherosklerose, Hypercholesterinämie, Hyperlipoproteinämie und ähnliche Erkrankungen.

Die Erfindung betrifft daher auch pharmazeutische Präparate auf Basis der Verbindungen der Formel V bzw. der entsprechenden Hydroxycarbonsäuren V', deren Salzen und Ester, sowie die Verwendung dieser Verbindungen zur Herstellung von Arzneimitteln, insbesondere zur Behandlung der Hypercholesterinämie.

Die Verbindungen der Formel V bzw. die entsprechenden Säuren, Salze oder Ester werden in verschiedenen Dosierungsformen verabreicht, vorzugsweise oral in Form von Tabletten, Kapseln oder Flüssigkeiten. Die tägliche Dosis bewegt sich je nach Körpergewicht und Konstitution des Patienten im Bereich von 3 mg bis 2500 mg, vorzugsweise jedoch im Dosisbereich 10 - 500 mg.

Die erfindungsgemäßen Verbindungen können als Laktone der allgemeinen Formel V, in Form der freien Säure V' oder in Form pharmazeutisch annehmbarer Salze oder Ester zur Anwendung kommen, und zwar gelöst oder suspendiert in pharmakologisch unbedenklichen organischen Lösungsmitteln wie ein- oder mehrwertigen Alkohohlen wie z.B. Ethanol oder Glycerin, in Triacetin, Ölen wie z.B. Sonnenblumenöl oder Lebertran, Ethern wie z.B. Diethylenglykoldimethylether oder auch Polyethern wie z.B. Polyethylenglykol oder auch in Gegenwart anderer pharmakologisch unbedenklicher Polymerträger wie z.B. Polyvinylpyrrolidon oder im Gemisch mit anderen pharmazeutisch annehmbaren Zustzstoffen wie Stärke, Cyclodextrin oder Polysacchariden. Ferner können die erfindungsgemäßen Verbindungen kombiniert werden mit Zusatzstoffen, die Gallensäure binden, insbesondere nicht toxische, basische Anionenaustauscherharze, die Gallensäuren in einer nichtresorbierbaren Form im Gastrointestinaltrakt binden. Die Salze der Hydroxycarbonsäuren können auch als wäßrige Lösung verarbeitet werden.

Die HMG-CoA-Reduktase-Aktivität wurde an folgendem Testsystem bestimmt:

Inhibierung der HMG-CoA-Reduktase-Aktivität an solubilisierten Enzympräparationen aus Ratten-Lebermikrosomen

Die HMG-CoA-Reduktase-Aktivität wurde an solubilisierten Enzympräparationen aus Lebermikrosomen von Ratten gemessen, die nach Umstellung im Tag-Nacht-Rhytmus mit Cholestyramin (ᴿCuemid) induziert wurden. Als Substrat diente (S,R) $^{14}$C-HMG-CoA, die Konzentration von NADPH wurde während der Inkubation durch ein regenerierendes System aufrecherhalten. Die Abtrennung von $^{14}$C-Mevalonat von Substrat und anderen Produkten (z.B. $^{14}$-C-HMG) erfolgte über Säulenelution, wobei das Eltuionsprofil jeder Einzelprobe ermittelt wurde. Auf die ständige Mitführung von $^{3}$H-Mevalonat wurde verzichtet, weil es sich bei der Bestimmung um die Realtivangabe der Hemmwirkung handelt. In einer Versuchsreihe wurde jeweils die enzymfreie Kontrolle, der enzymhaltige Normalansatz (= 100 %) und solche mit Präparatezusätzen zusammen behandelt. Jeder Einzelwert wurde als Mittelwert aus 3 Parallelproben gebildet. Die Signifikanz der Mittelwertsunterschiede zwischen präparatfreien und präparathaltigen Proben wurde nach dem t-Test beurteilt.

Nach der oben beschriebenen Methode wurden von den erfindungsgemäßen Verbindungen V im Vergleich zu den in der deutschen Patentanmeldung P 35 43 336.1 (= US-Patentanmeldung Nr. 900 848) beschriebenen Verbindungen A und B (A= 6(S)-{4,6-Dimethyl-2-[3-(4-fluorphenoxy)propyl]-phenoxymethyl}-3,4,5,6-tetrahydro-4(R)-hydroxy-2H-pyran-2-on; B= 3(R), 5(S)-Dihydroxy-6-{4,6-Dimethyl-2-[3-(4-fluorphenoxy)propyl]phenoxy}hexansäure Natriumsalz) z.B. folgende Hemmwerte auf die HMG-CoA-Reduktase ermittelt [$IC_{50}$-Wert (Mol/l) bedeutet die molare Konzentration der Verbindungen pro Liter, die für eine 50 %ige Hemmung erforderlich ist]:

| Verbindung | $IC_{50}$-Wert (Mol/l) |
|---|---|
| A | $1 \cdot 10^{-6}$ |
| B | $2 \cdot 10^{-7}$ |
| Va (Beispiel 4) | $3 \cdot 10^{-7}$ |
| Vc (Beispiel 6) | $\sim 10^{-4}$ |
| Vd (Beispiel 8) | $1 \cdot 10^{-6}$ |
| Ve (Beispiel 9) | 24 % Hemmung bei $10^{-7}$<br>37 % Hemmung bei $10^{-6}$ |

Beispiele: alle $^{1}$-NMR-Spektren (wenn nicht anders angegeben) in $CDCl_3$ mit einer Spur TMS als internem Standard

Herstellung von α,β-ungesättigten Lactonen III bzw. IV

Beispiel 1

6S-({[2-(4-Fluorphenoxy-3-propyl)]-4,6-dimethyl}phenoxymethyl)-5,6-dihydro-2H-pyran-2-on(IIIa)

Eine Lösung von 482 mg (1,2 mmol) des Hydroxylaktons 6(S)-{4,6-Dimethyl-2-[3-(4-fluorphenoxy)-propyl]phenoxymethyl}-3,4,5,6-tetrahydro-4(R)-hydroxy-2H-pyran-2-on (vgl. deutsche Patentanmeldung P 35 43 336.2 = US-Patentanmeldung Nr. 900 848 und Schema 2, Ia) in 10 ml trockenem Benzol wurde zur Lösung von 429 mg (1,8 mmol) des Burgess-Reagenz (E.M. Burgess et al., J. Org. Chem. 38, 26 (1973)) in 30 ml Benzol getropft. Das Reaktionsgemisch wurde 60 min auf 50 °C Innentemperatur erwärmt, wobei sich ein feiner weißer Feststoff abschied. Da DC-Kontrolle noch wenig Ausgangsmaterial anzeigte, wurde kurz mit Eis gekühlt, weitere 216 mg Burgess-Reagenz zugesetzt und noch 30 min auf 60 °C erwärmt. Bei Raumtemperatur wurden 10 ml Wasser zugegeben, 10 min gerührt und die Benzol-Phase abgetrennt. Die wäßrige Phase wurde noch 2mal mit Ether extrahiert, die vereinigten organischen Extrakte mit gesättigter Kochsalzlösung gewaschen, über Magnesiumsulfat getrocknet, filtriert und eingeengt. Chromatographie über wenig Kieselgel mit 40 % Essigester/60 % Petrolether gabe 426 mg (1,11 mmol, 92 % Ausbeute) α,β-ungesättiges Lacton IIIa als farblosen Feststoff, Schmp. 77 bis 78 °C. $R_f$ (Essigester/Cyclohexan 1:1) : Ia (0,28), IIIa (0,66) MS $\overline{(EI)}$:$C_{23}H_{25}O_4F$, 384 ($M^+$), 273

($M^+$-F-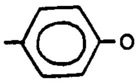-o.), 272 ($M^+$-F-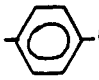-OH), 187, 135

$^1$H-NMR (270 MHz) : δ = 2,0 - 2,12 (qui, 2H, $CH_2$), 216 (s, 3H, $CH_3$), 2,18 (s, 3H, $CH_3$), 240 - 2,57 (dtd, 1H, Methylen-H), 2,67 - 2,80 (m, 3H), 3,92 - 4,00 (t, überlagert von d, 4H, Methylen-H), 4,68 - 4,80 (d qua, 1H, CH), 6,04 - 6,10 (ddd, 1H, =C-H), 6,8 - 7,0 (m, 3H, =C-H und 2 arom.-H)

Beispiel 2

6S({[2-(4-Fluorphenoxy-3-propyl)]-4,6-dimethyl}phenoxymethyl)-5,6-dihydro-2H-pryan-2-on (IIIa)

Zu einer Lösung von 128 mg (0,2 mmol) des Silyloxylaktons 6(S)-{4,6-Dimethyl-2-[3-(4-fluorphenoxy)-propyl]phenoxymethyl}-3,4,5,6-tetrahydro-4(R)-(t-butyl-diphenylsilyoxy)-2H-pyran-2-on (vgl. deutsche Patentanmeldung P 35 43 336.1 = US-Patentanmeldung Nr. 900 848 und Schema 2, I'a) in 7 ml Tetrahydrofuran wurden 190 mg (0,6 mmol) Tetrabutylammoniumfluorid-Trihydrat, dann 38 mg (0,2 mmol) p-Toluolsulfonsäure-Monohydrat gegeben. Man ließ 12 Stunden bei Raumtemperatur rühren, engte ein und chromatographierte den Rückstand über Kieselgel mit Cyclohexan/Essigester (1:1). Erhalten wurden 60,3 mg (0,157 mmol, 79% Ausbeute) IIIa, physikalische und Spektraldaten wie in Beispiel 1.

Beispiel 3

6S-[2-(4-Fluorbenzyl)-E-styryl]-5,6-dihydro-2H-pyran-2-on (IVb)

200 mg (0,61 mmol) des Hydroxylaktons (+)-E-6S-[2-(2-(4-Fluorbenzyl)phenyl-ethenyl]-4R-hydroxy-3,4,5,6-tetrahydro-2H-pyran-2-on (vgl. deutsche Patentanmeldung P 35 30 797.8 = US-Patentanmeldung Nr. 900 887 und Schema 3, IIb) und 365 mg (1,53 mmol) Burgess-Reagenz (E.M. Burgess et. al., J. Org. Chem 38, 26 (1973)) wurden in 230 ml trockenem Benzol gelöst. Die Lösung wurde 2 Stunden unter Stickstoff auf 60 °C erwärmt. Bei 25 °C wurden weitere 182 mg Burgess-Reagenz zugefügt und dann eine weitere Stunde bei 60 °C gerührt. DC (Cyclohexan/Essigester 1:1) zeigte vollständige Umsetzung zu IVb an [IIb ($R_f$ = 0,13), IVb (0,42)]. Das Lösungsmittel wurde im Vakuum entfernt und der Rückstand mit obigem Laufmittel über Kieselgel flash-chromatographiert. 130 mg (0,42 mmol, 69 % Ausbeute).
MS (EI) $C_{20}H_{17}O_2F$, 308 ($M^+$), 223, 196
$^1$H-NMR (270 MHz) : δ = 2,33 - 2,39 (m , 2H, $CH_2$), 3,96 (s, 2H, $CH_2$), 496 (qua mit Fernkopplung, 1H, CH), 5,98 (dt, 1H, olef.-H), 6,04 (dd, 1H, olef.-H), 6,78 - 7,22 (m, 9H, 8 arom.-H + 1 olef.-H), 7,40 (m, 1H, olef.-H)

Herstellung von Endprodukten

Beispiel 4

6S-({[2-(4-Fluorphenoxy-3-propyl)]-4,6-dimethyl}phenoxymethyl)-4R-acetylmercapto-3,4,5,6-tetrahydro-2H-pyran-2-on (Va, $R^8$ = Strukturelement VI)

Zu 115 mg (0,3 mmol) des ungesättigten Laktons IIIa (Beispiele 1, 2) wurden 36 µl (0,5 mmol) Thioessigsäure (frisch destilliert), dann 7 µl Triäthylamin gegeben. DC zeigte vollständige Reaktion nach 30 min an ($R_f$ bei 30 % Essigester/70 % Petrolether IIa 0,39, Va 0,48, Thioessigsäure 0,09). Filtration durch eine kurze Kieselgel-Säule gab 130 mg (0,28 mmol, 94 % Ausbeute) Addukt Va als zähes, farbloses Öl.
MS (FAB) : $C_{25}H_{29}SO_5F$, 461 ($M+H^+$), 348 ($M^+$ - F-$C_6H_4$-OH)
$^1$H-NMR (270 MHz) : δ = 2,0 - 2,1 (qui, 2H, $CH_2$), 2,1 - 2,2 (dt, 1H, Methylen-H); 2,23 (s, 3H, $CH_3$), 2,26 (s, 3H, $CH_3$), 2,36 (s, 3H, CO-$CH_3$), 2,44 - 2,56 (m, 1H, Methylen-H), 2,63 - 2,73 (ddd, 1H, Methylen-H), 2,76 (t, 2H, $CH_2$), 2,90 - 3,01 (ddd, 1H, Methylen-H), 3,88 - 4,0 (m, 4H, 2mal $OCH_2$), 4,15 (qui, 1H, CH), 4,77 (m, 1H, CH), 6,8 - 7,0 (m, 6H, arom.-H)

Beispiel 5

6S-[2-(4-Fluorbenzyl)-E-styryl]-4R-acetylmercapto-3,4,5,6-tetrahydro-2H-pyran-2-on (Vb, $R^8$ = Strukturelement VII)

Zur Lösung von 120 mg (0,39 mmol) des ungesättigten Laktons IV b (Beispiel 3) in 5 ml Toluol wurde die Lösung von 56 µl (0,78 mmol) Thioessigsäure in 1 ml Toluol, dann 27 µl (0,19 mmol) Triethylamin gegeben. Nach einer Stunde wurde das Lösungsmittel entfernt und der Rückstand mit Cyclohexan/Essigester (8:2) über Kieselgel gereinigt. Erhalten wurden 121 mg (0,31 mmol, 81 % Ausbeute) Vb als Öl.
$\overline{R_f}$ (Cyclohexan/Essigester 1:1) : IVb (0,38), Vb (0,47)
MS (EI): $C_{22}H_{21}O_3SF$ 384 ($M^+$), 341 ($M^+$ -$CH_3$-C=0), 308

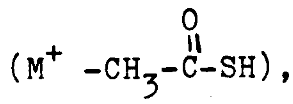

197, 109 (F-$C_6H_4$-$CH_2^{\oplus}$)
$^1$H-NMR (270 MHz) : $\delta$ = 2,0 - 2,2 (m, 2H, $CH_2$), 2,37 (s, 3H, $CH_3$), 2,61 (dd, 1H, Methylen-H), 2,87 (dd, 1H, Methylen-H), 3,86 (qui, 1H, CH), 4,03 (s, [sehr enges AB-System], 2H, $CH_2$), 5,12 (m, 1H, olef.-H), 685 (dd, 1H, olef.-H), 6,9 - 7,0 (m, 2H, arom.-H), 7,01 - 7,07 (m, 2H, arom.-H), 7,12 - 7,17 (m, 1H, arom.-H), 7,23 - 7,29 (m, 2H, arom.-H), 7,43 - 7,49 9m, 1H, arom.-H)

## Beispiel 6

6S-({[2-(4-Fluorphenoxy-3-propyl)]-4,6-dimethyl}phenoxymethyl-4R-benzylmercapto-3,4,5,6-tetrahydro-2H-pyran-2-on (Vc, $R^8$ = Strukturelement VI)

Zu 77 mg (0,2 mmol) des ungesättigten Laktons IIIa (Beispiele 1, 2) wurden 59 µl (0,5 mmol) Benzylmercaptan (frisch dest.), dann 14 µl (0,1 mmol) trock. Triethylamin gegeben. DC-Analyse nach 1 Stunde zeigte fast vollständige Reaktion an. Nach dem Stehen über Nacht bei 0°C unter Stickstoff war die Reaktion vollständig. $R_f$ bei 20 % Essigester/80 % Cyclohexan IIIa 0,19, Vc 0,39 Benzylmercaptan 0,82. Das Öl wurde direkt über eine kurze Kieselgelsäule flash-chromatographiert. Erhalten wurden 96,5 mg (0,19 mmol, 96 % Ausb.) Vc als zähes farbloses Öl.
MS (EI) $C_{30}H_{32}SO_4\overline{F}$, 508 ($M^+$), 396 ($M^+$ - F-$C_6H_4$-OH), 384 ($M^+$ - $C_6H_5SH$)
$^1$H-NMR (270 MHz) : $\delta$ = 1,98 - 2,1 (m, 2H, $CH_2$ und 1 Methylen-H), 2,23 (s, 3H, $CH_3$), 2,25 (s, 3H, $CH_3$), 2,25 - 2,36 (m, 1H, Methylen-H), 2,56 - 2,66 (ddd, 1H, Methylen-H), 2,73 (t, 2H, $CH_2$), 2,77 - 2,87 (dd, 1H, Methylen-H), 3,27 (qui, 1H, CH), 3,78 (enges AB-System, 2H, $SCH_2$), 3,87 (t, 2H, $OCH_2$), 3,94 (t, 2H, $OCH_2$), 4,85 (m, 1H, CH), 6,79 - 6, 87 (m, 4H, arom.-H), 6,9 - 7,0 (m, 2H, arom.-H) 7,28 - 7,35 (m, arom.-H)

## Beispiel 7

6-{[2-(4-Fluorphenoxy-3-propyl)]-4,6-dimethylphenoxy}-3R-benzylmercapto-5S-hydroxy-hexansäure-natriumsalz (V'c, NA-Salz $R^8$ = Strukturelement VI)

74 mg (0,145 mmol) Vc (Beispiel 6) wurden in 5 ml Ethanol gelöst und 1,37 ml 0,1 N Natronlauge zugegeben. Nach 2 Stunden wurde im Vakuum zur Trockne eingeengt. Der Rückstand wurde im vakuum zur Trockne eingeengt. Der Rückstand wurde 2mal mit Ether digeriert. Das Salz V'c wurde im Vakuum getrocknet; 69,4 mg (0,127 mmol, 88 % Ausbeute).
$^1$H-NMR ($D_2O$, 270 MHz, Bezugspunkt HOD $\delta$ = 4,80) : $\delta$= 1,2 - 1,56 (dt, 1H, Methylen-H), 1,55 - 1,85 (m, 2H, $CH_2$), 1,91 (s, 3H, $CH_3$), 2,01 (s, 3H, $CH_3$), 2,3 - 2,65 (m, 4H, 2$CH_2$), 3,0 - 3,45 (m, 3H), 3,45 - 3,6 (m, 4H), 3,96 - 4,11 (breit, 1H, CH), 6,46, 6,63 (m, 4H, arom.-H), 6,65 - 6,80 (m, 2H, arom.-H), 6,85 - 6,92 (m, 1H, arom.-H), 6,93 - 7,00 (t, 2H, arom.-H), 7,02 - 7,11 (t, 2H, arom.-H)

## Beispiel 8

6S-({[2-(4-Fluorphenoxy-3-propyl)]-4,6-dimethyl}phenoxymethyl)-4R-$\beta$-hydroxyethylmercapto-3,4,5,6-tetrahydro-2H-pyran-2-on (Vd, $R^8$ = Strukturelement VI)

Zu 87 mg (0,2227 mmol) des ungesättigten Laktons IIIa (Beispiele 1, 2) wurden 30 µl (0,43 mmol) Mercaptoethanol, dann 14 µl (0,1 mmol) Triethylamin zugesetzt. DC zeigte vollständige Reaktion nach einer Stunde an. Das Öl wurde direkt mit 50 % Essigester/50 % Petrolether über eine kurze Kieselgel-Säule

flash-chromatographiert. Überschüssiges Mercaptoethanol wird vor dem Produkt Vd (63,1 mg, 0,136 mmol, 60 % Ausbeutet, Öl) eluiert.
MS (EI) $C_{25}H_{31}SO_5F$, 462 ($M^+$), 384 ($M^+$ -$HOCH_2CH_2SH$), 350 ($M^+$ -$FC_6H_4$-OH)
$^1$H-NMR (270 MHz) δ = 1,76 (s, 1H, OH), 2,0 - 2,1 (qui, 2H, $CH_2$), 2,13 - 2,2 (t, 1H, Methylen-H), 2,23 (s, 3H, $CH_3$), 2,25 (s, 3H, $CH_3$), 2,38 - 2,50 (m, 1H, Methylen-H), 2,59 - 2,70 (ddd, 1H, Methylen-H), 2,71 - 2,80 (m, 4H, 2mal $CH_2$), 2,88 - 2,97 (dd, 1H, Methylen-H), 3,48 - 3,57 (qui, 1H, 4,94 (m, 1H, CH), 6,8 - 6,9 (m, 4H, arom.-H), 6,92 - 7,0 (m, 2H, arom.-H).

## Beispiel 9

6S-({[2-(4-Fluorphenoxy-3-propyl)]-4,6-dimethyl}phenoxymethyl)-4R-[(R)-2-amino-3-mercaptyl-propionsäure]-3,4,5,6-tetrahydro-2H-pyran-2-on (Ve, $R^8$ = Strukturelement VI)

Das Gemisch aus 77 mg (0,2 mmol) des ungesättigten Laktons IIIa (Beispiele 1, 2), 32 mg (0,3 mmol) Natriumcarbonat, 62 mg (0,4 mmol) L-Cystein-hydrochlorid wurde bei 25 °C in 1 ml absol. Ethanol gelöst. DC nach einer Stunde zeigte keine Reaktion an. Es wurden 50 µl (0,36 mmol) Triethylamin zugegeben, wobei sich sofort ein farbloser Niederschlag bildete. Das Lösungsmittel wurde abgezogen und der Rückstand in der Mindestmenge Chloroform/Methanol (7:3) gelöst. Das Rohrprodukt wurde mit Methylenchloride/Methanol (7:3) durch Kieselgel (Ø 2,5 cm, Höhe 13 cm) chromatographiert. Erhalten wurden 98,3 mg (0,195 mmol, 97 % Ausbeute) Ve als farbloses Pulver, schmp. (ohne Umkrist.) 175 - 176 °C (unter Zersetzung, Gelbfärbung, Gasen)
MS (FAB, NBA-Matrix): $C_{26}H_{32}NO_6SF$ 574 ($M + Na_3^+$), 550 ($M + NA_2^+$ - $H^+$), 528 (M + $Na^+$), 506 (M + $H^+$), 407, (M + $Na^+$ - F-$C_6H_4$O.)
IR (KBr) : 3600 - 3300 $cm^{-1}$ (breit, $NH_2$), 3300 - 3000 breit, -$NH_3^+$), 3000 - 2400 (sehr breit, $CO_2H$), 1740 (C = 0 von Lakton), 1510 (Aromat)
$^1$H-NMR (DMSO-$d_6$, 270 MHz) δ = 1,9 - 2,0 (qui, 2H, $CH_2$), 2,08 (dt, 1H, Methylen-H), 2,18 (s, 3H, $CH_3$), 2,20 (s, 3H, $CH_3$), 2,28 (dt, 1H, Methylen-H), 2,56 (dd, 1H, S-CH), 2,70 (t, 2H, $CH_2$), 2,86 (dd, 1H, Methylen-H), 2,92 (dd, 1H, S-CH), 3,11 (dd, 1H, Methylen-H), 3,2 - 3,5 (breit, 3H, $NH_3^+$), 3,37 (qui, 1H, CH), 3,56 (qui, 1H, CH), 3,85 - 3,9 (m, 2H, $OCH_2$), 3,96 (t, 2H, $OCH_2$), 4,83 - 4,92 (m, 1H, CH), 6,83 (s, 2H, arom.-H), 6,9 - 6,97 (m, 2H, arom.-H), 7,07 - 7,15 (m, 2H, arom.-H)

## Beispiel 10

6-{[2-(4-Fluorphenoxy-3-propyl)]-4,6-dimethylphenoxy-3R-[(R)-2-Amino-3-mercaptyl-natriumpropionat]-5S-hydroxy-hexansäure-natriumsalz (V'e, Na-Salz $R^8$ = Strukturelement (VI)

200 mg (0,393 mmol) Ve (Beispiel 9) wurden in 10 ml absol. Ethanol suspendiert. 3,93 ml 0,1 N Natronlauge wurden zugegeben, nach 10 min weitere 3,73 ml 0,1 N NaOH. Nach 30 min wurde das Lösungsmittel abgezogen. Der Rückstand wurde mit 3 mal 3 ml Ethanol gewaschen und im Vakuum getrocknet. Ausbeute 92 mg V'e.
$^1$H-NMR ($D_2O$, 270 MHz, Bezugspunkt HOD δ = 4,80) : δ = 1,75 - 1,95 (m, 2H, $CH_2$), 2,0 - 2,12 (qui, 2H, $CH_2$), 2,22 (s, 3H, $CH_3$), 2,26 (s, 3H, $CH_3$), 2,32 (dd, 1H, S-CH), 2,59 (dd, 1H, (S-CH), 2,69 - 2,83 (m, 3H, $CH_2$ und 1 Methylen-H), 2,9 - 3,0 (m, 1H, Methylen-H), 3,15 - 3,25 (m, 1H, Methylen-H), 3,36 (s, 5H, OH, $NH_3^+$, $CO_2H$), 3,42 (dd, 1H, CH), 3,78 (d, 2H, $OCH_2$), 4,05 (t, 2H, $OCH_2$), 4,15 (m, 1H, CH), 6,9 - 7,0 (m, 4H, arom.-H), 7,05 - 7,12 (t, 2H, arom.-H).

## Beispiel 11

6-{[2-(4-Fluorphenoxy-3-propyl)]-4,6-dimethylphenoxy-3R-β-hydroxyethylamino-5S-hydroxy-hexansäure-natriumsalz (V'f, Na-Salz $R^8$ = Strukturelement VI)

a) Zu 128 mg (0,3 mmol) des ungesättigten Laktons IIIa (Beispiele 1, 2) wurden 200 ml trockenes Dimethoxyethan und 180 µl (3,5 mmol) Ethanolamin gegeben. Nach 1 Stunde bei 25 °C war die Reaktion beendet.

$R_f$ bei 70 % Chloroform/30 % Methanol : IIIa 0,78, V'f-Ethanolamid 0,15. Das Reaktionsgemisch wurde mit dem gleichen Laufmittel über eine kurze Kieselgel-Säule filtriert. Erhalten wurden 139 mg V'f-Ethanolamid (0,275 mmol, 91 % Ausb.) eines blaßgelben Öls.
MS (FAB, 3-NBA-Matrix) $C_{27}H_{39}FN_2O_6$ 507 (M + $H^+$) MS (EI): 506 ($M^+$, sehr schwach), 487 ($M^+$ - $H_2O$ -

H.), 475 ($M^+$ -.$CH_2OH$), 445 ($M^+$ - $HOCH_2CH_2NH_2$), 4527 ("445" - $H_2O$), 414 ("475" - $HOCH_2CH_2NH_2$), 384 ($M^+$ - $2HOCH_2CH_2NH_2$), 316 ("427" - F-$C_6H_4O$.), 274 ($m^+$ - F-$C_6H_4$OH-2 $HNCH_2CH_2OH$)
$^1$H-NMR (270 MHz) $\delta$ = 1,6 - 1,8 (m, 2H, $CH_2$), 2,4 (qui, 2H, $CH_2$), 2,22 (s, 6H, 2x$CH_3$), 2,33 (dd, 1H, Methylen-H), 2,52 (dd, 1H, Methylen-H), 2,7 - 3,1 (m, 7H, 2x$CH_2$, 2xOH, Amin-H), 3,25 - 3,5 (m, 3H, $OCH_2$, CH), 3,6 - 3,8 (m, 6H, 3x$CH_2$), 3,92 (t, 2H, $OCH_2$), 4,18 (m, 1H, CH), 6,8 - 6,9 (m, 4H, arom.-H), 6,9 - 7,0 (t, 2H, arom.-H), 7,48 (t, 1H, Amid-H)
b) Die Lösung von 55 mg (0,11 mmol) V'f-Ethanolamid und 427 mg (10,6 mmol) NaOH in 1,1 ml Wasser und 1 ml Ethanol wurde 3 Stunden unter Rückfluß gekocht. Nach dem Abkühlen wurden 5,2 ml 2 N Salzsäure zugegeben (+ pH 8 - 9) und im Vakuum zur Trockne eingeengt.
$R_f$ bei 50 % Chloroform/50 % Methanol: V'f-Ethanolamid 0,16, V'f (als Carbonsäure) 0,34

Beispiel 12

6-{[2-(4-Fluorphenoxy-3-propyl)]-4,6-dimethyl}phenoxy-3R-benzylamino-5S-hydroxy-hexansäurenatriumsalz (V'g, Na-Salz, $R^8$ = Strukturelement VI)

a) Zu 77 mg (0,2 mmol) des ungesättigten Laktons IIIa (Beispiele 1, 2) wurden 44 µl (0,4 mmol) frisch destilliertes Benzylamin und 100 µl Benzol gegeben. Die Reaktionslösung wurde 12 Stunden auf 50 - 60° C erwärmt (nach 6 Stunden wurden weitere 200 µl Benzol zugesetzt). Flash-Chromatographie durch Kieselgel (∅ 4 cm, Höhe 10 cm) mit 1 l 75 % Essigester/25 % Petrolether, dann mit 99 % Essigester /1 % Triethylamin gab nach einem Vorlauf das reine Produkt V'g-benzylamid (starkes Tailing, 119 mg, 0,1999 mmol, 99 % Ausb.) als zähes, gelbliches Öl.
$R_f$ (100 % Essigester) : IIIa 0,9, V'g-benzylamid 0,44 MS (FAB, NBA-Matrix); $C_{37}H_{43}N_2O_4F$ 599 ($M + H^+$), 450 ($M + H^+$-F-$C_6H_4$-OH - $2H_2O$)
IR ($CHCl_3$) : 3600 - 3100 $cm^{-1}$ (OH und NH), 1645/1550 (Amid), 1506 (Aryl), 1210
$^1$H-NMR (270 MHz) $\delta$ = 1,65 - 1,76 (m, 2H, $CH_2$), 2,03 (qui, 2H, $CH_2$), 2,23 (s, 6H, 2x$CH_3$), 2,34 (dd, 1H, Methylen-H), 2,59 (dd, 1H, Methylen-H), 2,7 - 2,8 (m, 2H, $CH_2$), 3,30 (sext., 1H, CH), 3,65 (d, 2H, $NCH_2$), 3,74 (d, 1H, $NCH_2$), 3,90 (dd, 1H, $NCH_2$), 3,90 (t, 2H, $OCH_2$), 4,1 - 4,2 (m, 1H, CH), 4,42 (AB-Teil von ABX, 2H, $OCH_2$), 6,77 - 6,92 (m, 6H, arom.-H) 7,2 - 7,35 (m, 12H, 10 arom.-H und 2 NH)
b) Verseifung des Benzylamids von V'g wurde in analoger Weise wie in Beispiel 11 beschrieben durchgeführt. Man erhielt das Natriumsalz V'g.

Beispiel 13

6S-({[2-(4-Fluorphenoxy-3-propyl)]-4,6-dimethyl}phenoxymethyl)-4R-[(bis-ethoxycarbonyl)-methyl]-3,4,5,6-tetrahydro-2H-pyran-2-on (Vh, $R^8$ = Strukturelement VI)

Zur Lösung von 115,2 mg (0,3 mmol) IIIa (Beispiele 1, 2) und 58,6 mg (0,36 mmol) Malonsäurediethylester gibt man das Natriumhydrid, das nach 2maligem Waschen/Dekantieren von 20 mg einer 55 - 60 %igen NaH-Suspension in Öl mit Pentan zurückbleibt. Es wird 2 Stunden bei Raumtemperatur gerührt. Das Reaktionsgemisch wird mittels Filtration mit Cyclohexan/Essigester (2 : 1) durch Kieselgel gereinigt.
$R_f$ (Cyclohexan/Essigester 3 : 2) : Vh 0,49, Verunreinigung (Vh-Stereoisomers) 0,42, IIIa 0,32
Man erhält 115 mg (0,21 mmol, 70 % Ausbeute) Vh als farbloses Öl
MS (EI) : $C_{30}H_{37}FO_8$, 544 ($M^+$), 499 ($M^+$-$OCH_2CH_3$), 432 ($M^+$-F-$C_6H_4$OH), 387 ("432" - $OCH_2CH_3$), 271
$^1$H-NMR (270 MHz) $\delta$ = 1,25 (t, 3H, Ester, $CH_3$), 1,30 (t, 3H, Ester, $CH_3$), 1,82 (qua, 1H, CH), 2,05 (qui, 2H, $CH_2$, 2,24 (s, 6H, 2x$CH_3$), 2,25 - 2,40 (m, 2H, 2mal je ein Methylen-H), 2,48 (dd, 1H, Methylen-H), 2,75 (t, 2H, $CH_2$), 3,32 (d, 1H, CH), 3,88 - 4,0 (m, 4H, 2x$OCH_2$), 4,20 (qua, 2H, Ester-$CH_2$), 4,26 (qua, 2H, Ester-$CH_2$), 4,7 (m, 1H, CH), 6,8 - 6,88 (m, 4H, arom.-H), 6,9 - 7,0 (t, 2H, arom.-H)

Beispiel 14

6-{[2-(4-Fluorphenoxy-3-propyl)]-4,6-dimethylphenoxy}-3R-[(bis-natriumcarboxyl)-methyl]-5S-hydroxy-hexansäure-natriumsalz (V'h, Na-Salz $R^8$ = Strukturelement VI)

61 mg Vh (0,112 mmol) wurden in 3 ml Ethanol/5 Tropfen Chloroform gelöst und und 3,36 ml (3 Äquivalente) 0,1 N Natronlauge zugesetzt. Nach 6-stündigem Rühren wurden die flüchtigen Bestandteile abgezogen und der Rückstand 2mal mit Toluol im Hochvakuum eingeengt. DC zeigte vollständige Verseifung an zu V'h.

**Patentansprüche**

**Patentansprüche für folgende Vertragsstaaten : BE, CH, DE, FR, GB, IT, LI, LU, NL, SE**

**1.** 4(R)-substituierte 6(S)-Phenyloxymethyl-6(S)-β-Phenyläthyl- und 6(S)-β-Styryl-tetrahydropyran-2-one der allgemeinen Formel V

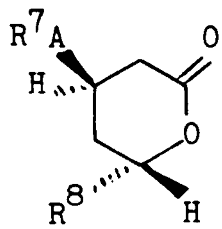

V

worin bedeuten:

A -S-, -NH-, $NR^7$ oder $-CHR^7-$

$R^7$

a) einen geradkettigen oder verzweigten Alkylrest mit 1 - 3 Kohlenstoffatomen, der substituiert sein kann

aa) mit einer Hydroxygruppe

ab) mit einer Amino- oder Ammonium-Gruppe

ac) mit einer Carboxylgruppe $CO_2H$, deren Methyl-, Ethyl- oder Benzylester, Methyl- oder Dimethylamid, Natrium-, Kalium- oder Ammonium-Salz

ad) mit einem Phenylrest

ae) mit 1 bis 3 Halogenatomen

b) eine Alkanoylgruppe

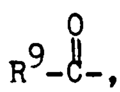,

worin $R^9$ ein geradkettiger oder verzweigter Alkylrest mit 1 bis 4 C-Atomen ist, der substituiert sein kann mit den unter aa) bis ae) angegebenen Gruppen

c) eine Carboxylgruppe, deren Methyl-, Ethyl- oder Benzylester, Methyl- oder Dimethylamid, Natrium-, Kalium- oder Ammonium-Salz

d) Wasserstoff

$R^8$ die Strukturelemente der Formeln VI oder VII

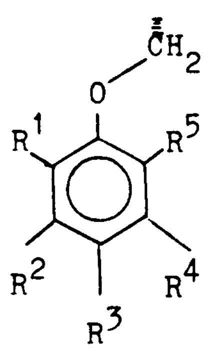

VI

oder

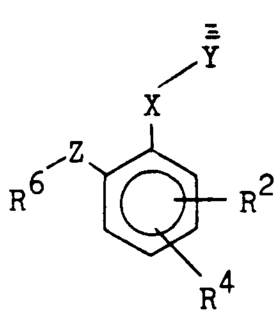

VII

worin bedeuten

X-Y einen Rest der Formel trans -CH=CH- oder $-CH_2-CH_2-$

Z eine $-CH_2-$ oder $-CH_2-CH_2-$Gruppe

$R^1$, $R^5$ gleich oder verschieden sind und

a) Wasserstoff oder Halogen

b) Cycloalkyl mit 4 bis 8 C-Atomen oder einen Phenylrest, der im Kern 1- bis 3-fach substituiert sein kann mit Halogen, Trifluormethyl und/oder Alkyl oder Alkoxy mit je 1 bis 4 C-Atomen oder

c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1 bis 3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettig oder verzweigten Alkenyloxy- oder Alkinyloxyresten mit 3 bis 6 Kohlenstoffatomen,

β) Halogen, Hydroxy, Cycloalkyl mit 3 bis 7 C-Atomen, unsubstituierten Phenyl-, α- oder β-Thienylresten, oder Phenyl-, α- oder β-Thienylresten, welche ihrerseits im Kern 1- bis 3-fach substituiert sind mit Halogen, Trifluormethyl und/oder Alkyl oder Alkoxy mit 1 bis 4 C-Atomen,

γ) unsubstituierten Phenoxy-, Benzyloxy-, α- oder β-Thienyloxyresten, oder Phenoxy-, Benzyloxy-, α- oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen.

δ) der Gruppe

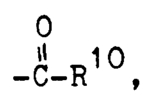

wobei $R^{10}$ bedeutet: einen geradkettigen oder verzweigten Alkyl- oder Alkenylrest mit bis zu 8 Kohlenstoffatomen, oder einen Cycloalkyl oder Cycloalkenylrest mit jeweils 3 bis 8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1 bis 4 C-Atomen, oder einen Pyridylrest,

$R^2$ und $R^4$, gleich oder verschieden sind und Wasserstoff, Alkyl mit 1 bis 4 C-Atomen, Halogen oder Alkoxy mit 1 bis 4 C-Atomen bedeuten, und

$R^3$ Wasserstoff, Alkyl oder Alkenyl mit bis zu 4 C-Atomen Halogen oder Alkoxy mit 1 bis 4 C-Atomen ist.

$R^6$ einen cycloaliphatischen Kohlenwasserstoffrest mit 3 bis 7 Kohlenstoffatomen, einen Phenylrest, der im Kern 1- bis 3-fach substituiert sein kann mit Halogen, Trifluormethyl, Alkyl oder Alkoxy mit je 1 bis 6 C-Atomen oder mit Hydroxymethyl, oder einen Furyl-, Thienyl- oder Pyridylrest, wobei die heteroaromatischen Rest 1- bis 2-fach substituiert sein können mit Halogen, Trifluormethyl, Alkyl oder Alkoxy mit je 1 bis 6 C-Atomen, sowie die entsprechenden offenkettigen Hydroxycarbonsäuren der Formel V'

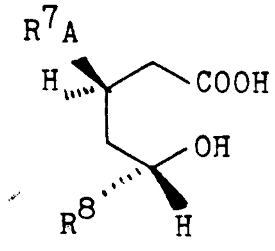

V'

worin A, $R^7$ und $R^8$ die zu Formel V angegebenen Bedeutungen haben, deren phamakologisch verträgliche Salze mit Basen und deren pharmakologisch verträgliche Ester.

**2.** Verbindungen gemäß Anspruch 1, dadurch gekennzeichnet, daß in den allgemeinen Formeln V bzw. V′

A -S-

$R^7$

a) einen geradkettigen oder verzweigten Alkylrest mit 1 bis 2 Kohlenstoffatomen, der substituiert sein kann mit

aa) einer Hydroxygruppe

ab) einer Amino- oder Ammonium-Gruppe

ac) einer Carboxylgruppe deren Methyl-, Ethyl- oder Benzylester, Methyl- oder Dimethylamid, Natrium-, Kalium- oder Ammoniumsalz

ad) einem Phenylrest

ae) einem Fluor- oder Chloratom

b) eine Alkanoylgruppe

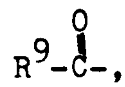

worin $R^9$ ein Methyl- oder Ethylrest ist, der substituiert sein kann mit einer Hydroxygruppe oder 1 bis 3 Fluoratomen

c) eine Carboxylgruppe, deren Methyl- oder Ethylester, Methyl- oder Dimethylamid, Natrium-, Kalium- oder Amminiumsalz,

d) Wasserstoff

$R^8$ die Strukturelemente der Formeln VI oder VII

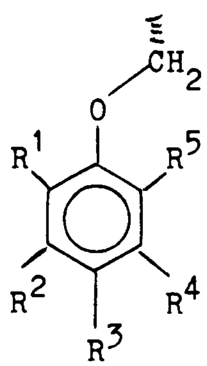

VI

oder

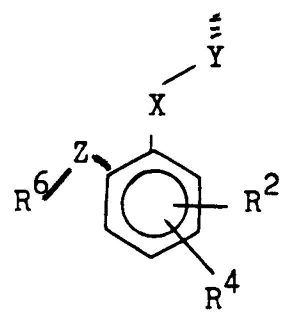

VII

worin

X-Y einen Rest der Formel trans -CH = CH- und

Z eine $CH_2$-Gruppe darstellen,

$R^1$, $R^5$ gleich oder verschieden sind und

a) Wasserstoff oder Chlor,

b) einen geradkettigen oder verzweigten Alkylrest mit 1 bis 6 Kohlenstoffatomen, wobei der Alkylrest seinerseits 1- bis 2-fach substituiert sein kann mit Phenyl- oder Phenoxy-Resten, die ihrerseits im Kern substituiert sein können mit Fluor oder Chlor, darstellen.

$R^2$, $R^4$ gleich oder verschieden sind und Wasserstoff, Methyl, Ethyl, Fluor oder Chlor sind,

$R^3$ Wasserstoff, Methyl oder Chlor,

$R^6$ einen cycloaliphatischen Kohlenwasserstoffrest mit 5 bis 6 C-Atomen, einen Phenylrest, der im Kern substituiert sein kann mit Fluor, Chlor, Trifluormethyl, Methyl, Ethyl, Methoxy, Ethoxy, darstellen, bedeuten.

**3.** Verbindungen gemäß Anspruch 1, dadurch gekennzeichnet, daß in den allgemeinen Formeln V bzw. V′

A -S-

$R^7$

a) einen Alkylrest mit 1 oder 2 Kohlenstoffatomen, der substituiert sein kann mit

aa) einer Hydroxygruppe

ab) einer Amino- oder Ammonium-Gruppe

ac) einer Carboxylgruppe, deren Methylester, Natrium-, Kalium- oder Ammonium-Salze

ad) einem Phenylrest

ae) einem Fluoratom

b) eine Acetyl- oder Trifluoracetylgruppe

$R^8$ die Strukturelemente der Formeln VI oder VII

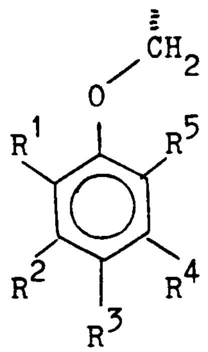

oder

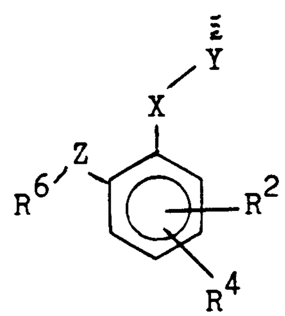

VI VII

worin

X-Y ein Rest der Formel trans -CH = CH- und

Z eine $-CH_2$-Gruppe sind,

$R^1$, $R^5$ gleich oder verschieden sind und

a) Chlor,

b) geradkettiger oder verzweigter Alkylrest mit 1 bis 3 C-Atomen, wobei der Alkylrest seinerseits 1 bis 2-fach substituiert sein kann mit Phenyl- oder Phenoxyresten, die ihrerseits im Kern substituiert sein können mit Fluor, und

$R^2$, $R^4$ Wasserstoff sind

$R^3$ Chlor oder Methyl und

$R^6$ Cyclohexyl-, unsubstituierter Phenylrest oder Phenylrest substituiert mit Chlor oder Fluor sind, bedeuten.

**4.** Verfahren zur Herstellung der Verbindungen gemäß Anspruch 1, dadurch gekennzeichnet, daß man

a) Verbindungen der allgemeinen Formeln I oder II

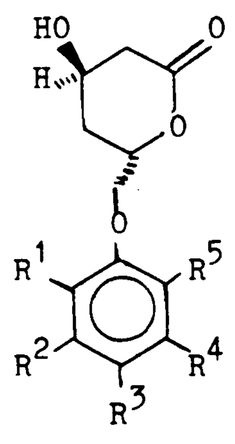

I

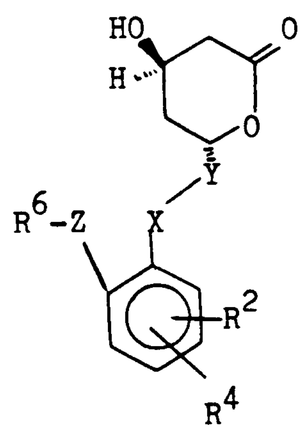

II

worin $R^1$, $R^2$, $R^3$, $R^4$, $R^5$, $R^6$, X, Y, Z die zur Formel V angegebene Bedeutung haben, mit einem

wasserabspaltenden Reagenz, zu den α, β-ungesättigten Laktonen der allgemeinen Formeln III oder IV umsetzt

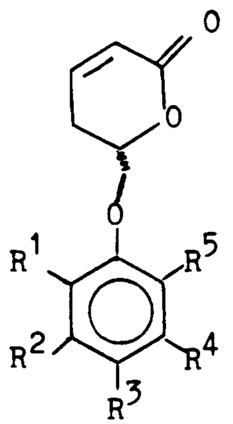

III

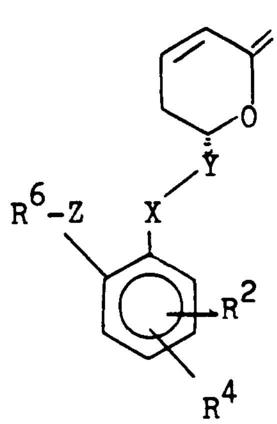

IV

oder die Vorstufen der Formeln I′ und II′

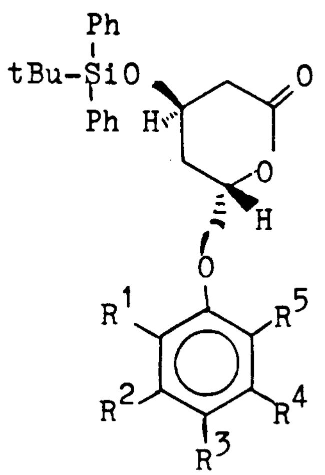

I'

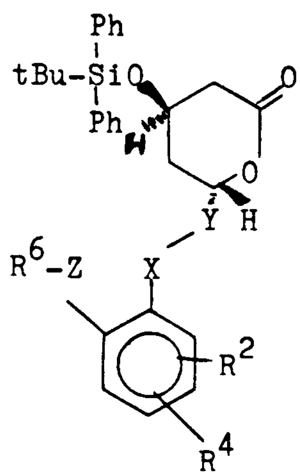

II'

worin $R^1$ bis $R^6$, X, Y und Z die zu Formel V angegebenen Bedeutungen haben, durch Desiloxylierung in die α,β-ungesättigten Lactone der Formeln III oder IV umsetzt und
b) die α,β-ungesättigten Lactone der Formeln III oder IV mit nucleophilen Verbindungen $R^7AH$, zu Verbindungen der Formel V, worin $R^7$ und A die zu Formel V angegebenen Bedeutungen haben und $R^8$ das Strukturelement VI, worin $R^1$ bis $R^5$ die angegebenen Bedeutungen haben, oder das Strukturelement VII, worin $R^2$, $R^4$ und $R^6$ die angegebenen Bedeutungen haben und X-Y die trans CH = CH-Gruppe darstellt, bedeutet, umsetzt und
gegebenenfalls eine erhaltene Verbindung V, worin X-Y die trans CH = CH-Gruppe darstellt, hydriert zu einer Verbindung V, worin X-Y die $CH_2$-CH-Gruppe darstellt,
gegebenenfalls eine erhaltene Verbindung der Formel V in die offenkettige Hydroxycarbonsäure der Formel V′ oder deren Ester oder Salz überführt, oder
gegebenenfalls ein Salz oder einen Ester in die freie Hydroxycarbonsäure überführt.

**5.** Pharmazeutische Präparate enthaltend eine Verbindung gemäß Anspruch 1.

**6.** Verwendung einer Verbindung gemäß Anspruch 1 zur zur Herstellung von Arzneimitteln für die Prophylaxe und Therapie der Hypercholesterinämie.

**7.** α,β-ungesättigte Lactone der Formel III

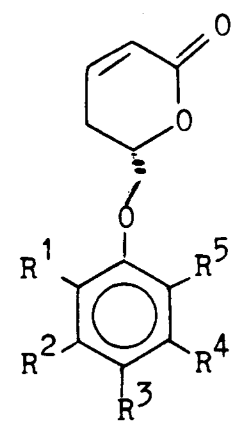

III

worin $R^1$ bis $R^5$ die in Anspruch 1 angegebenen Bedeutungen haben.

**8.** $\alpha,\beta$-ungesättigte Lactone der Formel IV

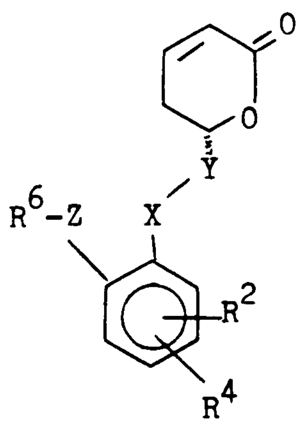

IV

worin $R^2$, $R^4$, $R^6$, X, Y und Z die in Anspruch 1 angegebenen Bedeutungen haben.

**Patentansprüche für folgende Vertragsstaaten : AT, ES, GR**

**1.** Verfahren zur Herstellung von 4(R)-substituierte 6(S)-Phenyloxymethyl-, 6(S)-$\beta$-Phenyläthyl- und 6(S)-$\beta$-Styryl-tetrahydropyran-2-onen der allgemeinen Formel V

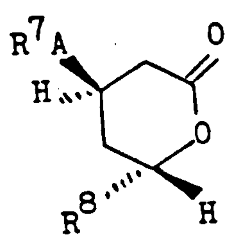

V

worin bedeuten:

A -S-, -NH-, $NR^7$ oder $-CHR^7-$

$R^7$

a) einen geradkettigen oder verzweigten Alkylrest mit 1 - 3 Kohlenstoffatomen, der substituiert sein kann

aa) mit einer Hydroxygruppe

ab) mit einer Amino- oder Ammonium-Gruppe

ac) mit einer Carboxylgruppe $CO_2H$, deren Methyl-, Ethyl- oder Benzylester, Methyl- oder Dimethylamid, Natrium-, Kalium- oder Ammonium-Salz

ad) mit einem Phenylrest

ae) mit 1 bis 3 Halogenatomen

b) eine Alkanoylgruppe

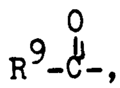

worin $R^9$ ein geradkettiger oder verzweigter Alkylrest mit 1 bis 4 C-Atomen ist, der substituiert sein kann mit den unter aa) bis ae) angegebenen Gruppen

c) eine Carboxylgruppe, deren Methyl-, Ethyl- oder Benzylester, Methyl- oder Dimethylamid, Natrium-, Kalium- oder Ammonium-Salz

d) Wasserstoff

$R^8$ die Strukturelemente der Formeln VI oder VII

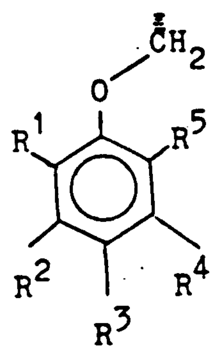

oder

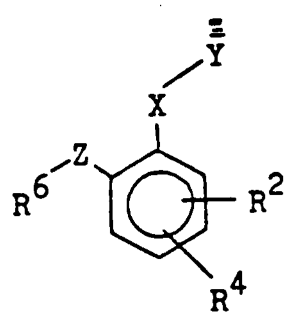

VI VII

worin bedeuten

X-Y einen Rest der Formel trans $-CH=CH-$ oder $-CH_2-CH_2-$

Z eine $-CH_2-$ oder $-CH_2-CH_2-$Gruppe

$R^1$, $R^5$ gleich oder verschieden sind und

a) Wasserstoff oder Halogen

b) Cycloalkyl mit 4 bis 8 C-Atomen oder einen Phenylrest, der im Kern 1- bis 3-fach substituiert sein kann mit Halogen, Trifluormethyl und/oder Alkyl oder Alkoxy mit je 1 bis 4 C-Atomen oder

c) einen geradkettigen oder verzweigten Alkylrest mit 1 bis 18 Kohlenstoffatomen oder einen geradkettigen oder verzweigten Alkenylrest mit 2 bis 18 Kohlenstoffatomen bedeuten, wobei die Alkyl- bzw. Alkenylreste ihrerseits 1 bis 3-fach substituiert sein können mit

α) geradkettigen oder verzweigten Alkoxyresten mit bis zu 10 Kohlenstoffatomen oder Cycloalkoxyresten mit 3 bis 7 Kohlenstoffatomen oder geradkettig oder verzweigten Alkenyloxy- oder Alkinyloxyresten mit 3 bis 6 Kohlenstoffatomen,

β) Halogen, Hydroxy, Cycloalkyl mit 3 bis 7 C-Atomen, unsubstituierten Phenyl-, α- oder β-Thienylresten, oder Phenyl-, α- oder β-Thienylresten, welche ihrerseits im Kern 1- bis 3-fach substituiert sind mit Halogen, Trifluormethyl und/oder Alkyl oder Alkoxy mit 1 bis 4 C-Atomen,

γ) unsubstituierten Phenoxy-, Benzyloxy-, α- oder β- oder β-Thienyloxyresten, welche ihrerseits im Kern 1- bis 3-fach substituiert sind mit Halogen, Trifluormethyl und/oder Alkyl oder Alkoxy mit 1 bis 4 Kohlenstoffatomen.

δ) der Gruppe

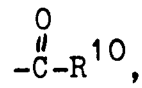

wobei $R^{10}$ bedeutet: einen geradkettgien oder verzweigten Alkyl- oder Alkenylrest mit bis zu 8 Kohlenstoffatomen, oder einen Cycloalkyl oder Cycloalkenylrest mit jeweils 3 bis 8 C-Atomen, oder einen unsubstituierten Phenylrest oder einen Phenylrest, welcher seinerseits im Kern 1- bis 3-fach substituiert ist mit Halogen, Trifluormethyl und/oder Alkyl oder Alkoxy mit 1 bis 4 C-Atomen, oder einen Pyridylrest,

$R^2$ und $R^4$, gleich oder verschieden sind und Wasserstoff, Alkyl mit 1 bis 4 C-Atomen, Halogen oder Alkoxy mit 1 bis 4 C-Atomen bedeuten, und

$R^3$ Wasserstoff, Alkyl oder Alkenyl mit bis zu 4 C-Atomen Halogen oder Alkoxy mit 1 bis 4 C-Atomen ist.

$R^6$ einen cycloaliphatischen Kohlenwasserstoff mit 3 bis 7 Kohlenstoffatomen, einen Phenylrest, der im Kern 1- bis 3-fach substituiert sein kann mit Halogen, Trifluormethyl, Alkyl oder Alkoxy mit je 1 bis 6 C-Atomen oder mit Hyroxymethyl, oder einen Furyl-, Thienyl- oder Pyridylrest, wobei die heteroaromatischen Reste 1- bis 2-fach substituiert sein können mit Halogen, Trifluormethyl, Alkyl oder Alkoxy mit je 1 bis 6 C-Atomen, sowie die entsprechenden offenkettigen Hydroxycarbonsäuren der Formel V′

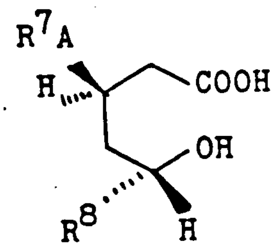

V'

worin A, $R^7$ und $R^8$ die zu Formel V angegebenen Bedeutungen haben, von deren phamakologisch verträglichen Salzen mit Basen und von deren pharmakologisch verträglichen Estern, dadurch gekennzeichnet, daß man

a) Verbindungen der allgemeinen Formeln I oder II

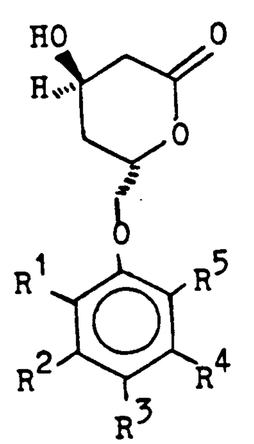

I

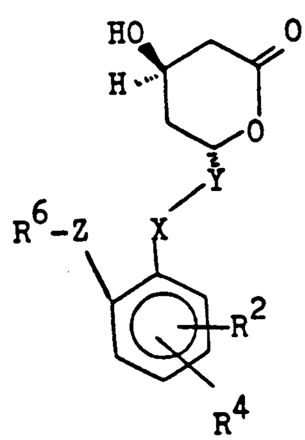

II

worin $R^1$, $R^2$, $R^3$, $R^4$, $R^5$, $R^6$, X, Y, Z die zur Formel V angegebene Bedeutung haben, mit einem wasserabspaltenden Reagenz, zu den $\alpha,\beta$-ungesättigten Laktonen der allgemeinen Formeln III oder IV umsetzt

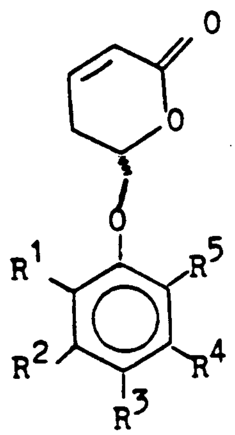

III

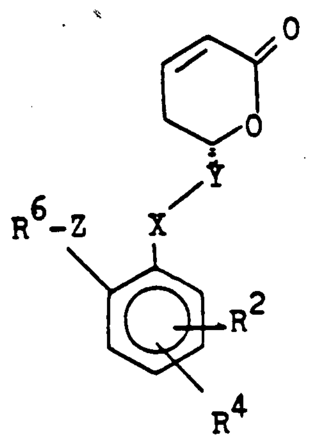

IV

oder die Vorstufen der Formeln I′ und II′

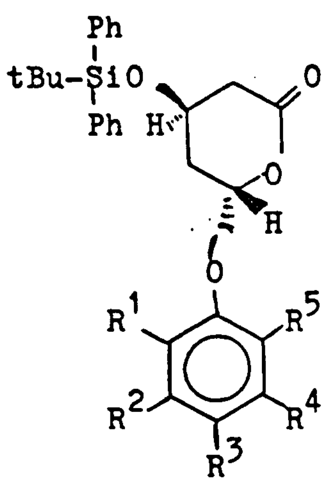

I'

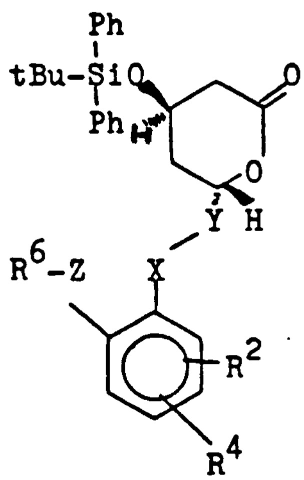

II'

worin $R^1$ bis $R^6$, X, Y und Z die zu Formel V angegebenen Bedeutungen haben, durch Desiloxylierung in die $\alpha,\beta$-ungesättigten Lactone der Formeln III oder IV umsetzt und

b) die $\alpha,\beta$-ungesättigten Lactone der Formeln III oder IV mit nucleophilen Verbindungen $R^7AH$, zu Verbindungen der Formel V, worin $R^7$ und A die zu Formel V angegebenen Bedeutungen haben und $R^8$ das Strukturelement VI, worin $R^1$ bis $R^5$ die angegebenen Bedeutungen haben, oder das Strukturelement VII, worin $R^2$, $R^4$ und $R^6$ die angegebenen Bedeutungen haben und X-Y die trans CH = CH-Gruppe darstellt, bedeutet, umsetzt und

gegebenenfalls eine erhaltene Verbindung V, worin X-Y die trans CH = CH-Gruppe darstellt, hydriert zu einer Verbindung V, worin X-Y die $CH_2$-$CH_2$-Gruppe darstellt, gegebenenfalls eine erhaltene Verbindung der Formel V in die offenkettige Hydroxycarbonsäure der Formel V′ oder deren Ester oder Salz überführt, oder gegebenenfalls ein Salz oder einen Ester in die freie Hydroxycarbonsäure überführt.

**2.** Verfahren gemäß Anspruch 1, dadurch gekennzeichnet, daß man eine Verbindung der allgemeinen Formel V bzw. V′, worin

A -S-

$R^7$

a) einen geradkettigen oder verzweigten Alkylrest mit 1 bis 2 Kohlenstoffatomen, der substituiert sein kann mit

aa) einer Hydroxygruppe

ab) einer Amino- oder Ammonium-Gruppe

ac) einer Carboxylgruppe deren Methyl-, Ethyl- oder Benzylester, Methyl- oder Dimethylamid, Natrium-, Kalium- oder Ammoniumsalz

ad) einem Phenylrest

ae) einem Fluor- oder Chloratom

b) eine Alkanoylgruppe

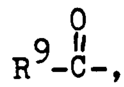

worin $R^9$ ein Methyl- oder Ethylrest ist, der substituiert sein kann mit einer Hydroxygruppe oder 1 bis 3 Fluoratomen

c) eine Carboxylgruppe, deren Methyl- oder Ethylester, Methyl- oder Dimethylamid, Natrium-, Kalium- oder Amminiumsalz,

d) Wasserstoff

$R^8$ die Strukturelemente der Formeln VI oder VII

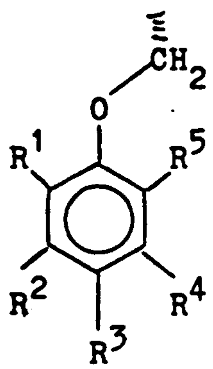

oder

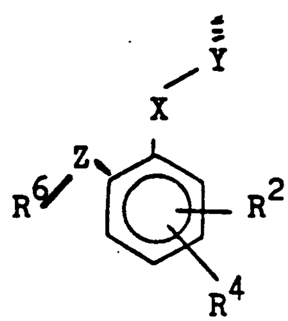

VI VII

worin

X-Y einen Rest der Formel trans -CH = CH- und

Z eine $CH_2$-Gruppe darstellen,

$R^1$, $R^5$ gleich oder verschieden sind und

a) Wasserstoff oder Chlor,

b) einen geradkettigen oder verzweigten Alkylrest mit 1 bis 6 Kohlenstoffatomen, wobei der Alkylrest seinerseits 1- bis 2-fach substituiert sein kann mit Phenyl- oder Phenoxy-Resten, die ihrerseits im Kern substituiert sein können mit Fluor oder Chlor, darstellen,

$R^2$, $R^4$ gleich oder verschieden sind und Wasserstoff, Methyl, Ethyl, Fluor oder Chlor sind,

$R^3$ Wasserstoff, Methyl oder Chlor,

$R^6$ einen cycloaliphatischen Kohlenwasserstoff mit 5 bis 6 C-Atomen, einen Phenylrest, der im Kern substituiert sein kann mit Fluor, Chlor, Trifluormethyl, Methyl, Ethyl, Methoxy, Ethoxy, darstellen, bedeuten, oder deren Ester oder Salze herstellt.

**3.** Verfahren gemäß Anspruch 1, dadurch gekennzeichnet, daß man eine Verbindung der allgemeinen Formel V bzw. V′ worin

A -S-

$R^7$

a) einen Alkylrest mit 1 oder 2 Kohlenstoffatomen, der substituiert sein kann mit

aa) einer Hydroxygruppe

ab) einer Amino- oder Ammonium-Gruppe

ac) einer Carboxylgruppe, deren Methylester, Natrium-, Kalium- oder Ammonium-Salze

ad) einem Phenylrest

ae) einem Fluoratom

b) eine Acetyl- oder Trifluoracetylgruppe

$R^8$ die Strukturelemente der Formeln VI oder VII

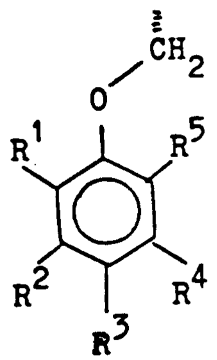

VI

oder

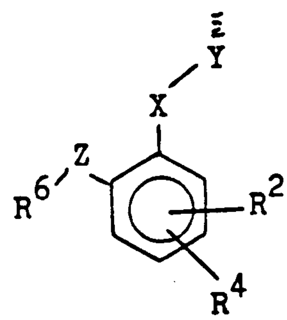

VII

worin

X-Y ein Rest der Formel trans -CH = CH- und

Z eine $-CH_2$-Gruppe sind,

$R^1$, $R^5$ gleich oder verschieden sind und

a) Chlor,

b) geradkettiger oder verzweigter Alkylrest mit 1 bis 3 C-Atomen, wobei der Alkylrest seinerseits 1 bis 2-fach substituiert sein kann mit Phenyl- oder Phenoxyresten, die ihrerseits im Kern substituiert sein können mit Fluor, und

$R^2$, $R^4$ Wasserstoff sind

$R^3$ Chlor oder Methyl und

$R^6$ Cyclohexyl-, unsubstituierter Phenylrest oder Phenylrest substituiert mit Chlor oder Fluor sind, bedeuten, oder deren Ester oder Salze herstellt.

**4.** Verfahren zur Herstellung eines pharmazeutischen Präparates, dadurch gekennzeichnet, daß man eine Verbindung erhältlich gemäß Verfahren nach Anspruch 1 in eine geeignete Darreichungsform überführt.

**5.** Verfahren gemäß Anspruch 1, dadurch gekennzeichnet, daß man die Zwischenstufe der Formel III

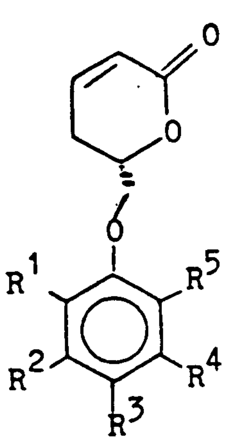

III

worin $R^1$ bis $R^5$ die zu Formel V in Anspruch 1 angegebenen Bedeutungen haben isoliert.

**6.** Verfahren gemäß Anspruch 1, dadurch gekennzeichnet, daß man die Zwischenstufe der Formel IV

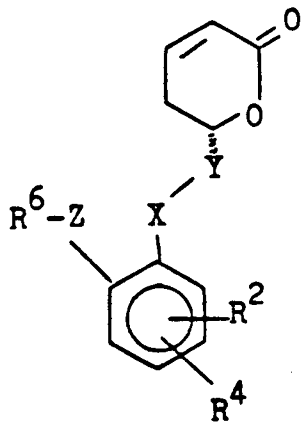

IV

worin $R^2$, $R^4$, $R^6$, X, Y und Z die zu Formel V in Anspruch 1 angegebenen Bedeutungen haben, isoliert.

**Claims**
**Claims for the following Contracting States : BE, CH, DE, FR, GB, IT, LI, LU, NL, SE**

**1.** A 4(R)-substituted 6(S)-phenyloxymethyl-, 6(S)-$\beta$-phenylethyl-and 6(S)-$\beta$-styryl-tetrahydropyran-2-one of the formula V

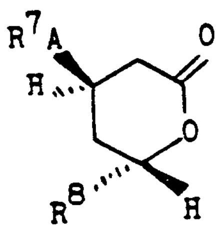

V

in which:

A denotes -S-, -NH-, $NR^7$ or $-CHR^7$-

$R^7$

a) denotes a straight-chain or branched alkyl radical which has 1 - 3 carbon atoms and can be substituted
aa) by a hydroxyl group
ab) by an amino or ammonium group
ac) by a carboxyl group $CO_2H$ or the methyl, ethyl or benzyl ester or methyl- or dimethylamide or sodium, potassium or ammonium salt thereof
ad) by a phenyl radical
ae) by 1 to 3 halogen atoms

b) denotes an alkanoyl group

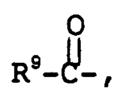

in which $R^9$ is a straight-chain or branched alkyl radical which has 1 to 4 carbon atoms and can be substituted by the groups mentioned under aa) to ae)

c) denotes a carboxyl group, the methyl, ethyl or benzyl ester, or methyl- or dimethylamide, or sodium, potassium or ammonium salt thereof, or

d) denotes hydrogen

$R^8$ denotes the structural elements of the formulae VI or VII

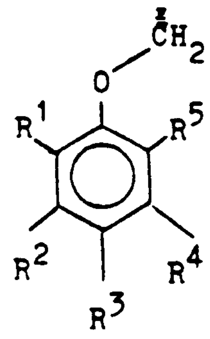

or

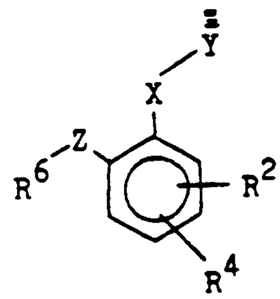

VI VII

in which

X-Y denotes a radical of the formula trans $-CH=CH-$ or $-CH_2-CH_2-$

Z denotes a $-CH_2-$ or $-CH_2-CH_2-$ group

$R^1$ and $R^5$ are identical or different and

a) denote hydrogen or halogen

b) denote cycloalkyl with 4 to 8 carbon atoms or a phenyl radical, which can be mono-, di- or tri- substituted in the nucleus by halogen, trifluoromethyl and/or alkyl or alkoxy with in each case 1 to 4 carbon atoms, or

c) denote a straight-chain or branched alkyl radical with 1 to 18 carbon atoms or a straight-chain or branched alkenyl radical with 2 to 18 carbon atoms, it being possible for the alkyl and alkenyl radicals in turn to be mono-, di- or trisubstituted by

α) straight-chain or branched alkoxy radicals with up to 10 carbon atoms or cycloalkoxy radicals with 3 to 7 carbon atoms or straight-chain or branched alkenyloxy or alkynyloxy radicals with 3 to 6 carbon atoms,

β) halogen, hydroxyl, cycloalkyl with 3 to 7 carbon atoms, unsubstituted phenyl or α- or β-thienyl radicals, or phenyl or α- or β-thienyl radicals which are in turn mono-, di- or trisubstituted in the nucleus by halogen, trifluoromethyl and/or alkyl or alkoxy with 1 to 4 carbon atoms,

γ) unsubstituted phenoxy, benzyloxy or α- or β-thienyloxy radicals, or phenoxy, benzyloxy or α- or β-thienyloxy radicals which are in turn mono-, di- or trisubstituted in the nucleus by halogen, trifluoromethyl and/or alkyl or alkoxy with 1 to 4 carbon atoms,

δ) the group

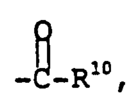

in which $R^{10}$ denotes: a straight-chain or branched alkyl or alkenyl radical with up to 8 carbon atoms, or a cycloalkyl or cycloalkenyl radical with in each case 3 to 8 carbon atoms, or an unsubstituted phenyl radical or a phenyl radical which is in turn mono-, di- or trisubstituted in the nucleus by halogen, trifluoromethyl and/or alkyl or alkoxy with 1 to 4 carbon atoms, or a pyridyl radical,

$R^2$ and $R^4$ are identical or different and denote hydrogen, alkyl with 1 to 4 carbon atoms, halogen or alkoxy with 1 to 4 carbon atoms, and

$R^3$ denotes hydrogen, alkyl or alkenyl with up to 4 carbon atoms, halogen or alkoxy with 1 to 4 carbon atoms, and

$R^6$ denotes a cycloaliphatic hydrocarbon radical with 3 to 7 carbon atoms, a phenyl radical which can be mono-, di- or trisubstituted in the nucleus by halogen, trifluoromethyl, alkyl or alkoxy with in each case 1 to 6 carbon atoms or hydroxymethyl, or a furyl, thienyl or pyridyl radical, it being possible for the heteroaromatic radicals to be mono- or disubstituted by halogen, trifluoromethyl or alkyl or alkoxy

with in each case 1 to 6 carbon atoms,
and the corresponding open-chain hydroxycarboxylic acid of the formula V'

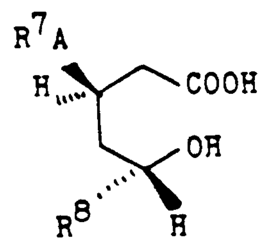

V'

in which A, $R^7$ and $R^8$ have the meanings given in the case of formula V, or a pharmacologically acceptable salt thereof with a base or a pharmacologically acceptable ester thereof.

**2.** A compound as claimed in claim 1, in which, in the formula V or V',

A denotes -S-

$R^7$

a) denotes a straight-chain or branched alkyl radical which has 1 or 2 carbon atoms and can be substituted by
aa) a hydroxyl group
ab) an amino or ammonium group
ac) a carboxyl group, or the methyl, ethyl or benzyl ester, or methyl- or dimethylamide, or sodium, potassium or ammonium salt thereof
ad) a phenyl radical
ae) a fluorine or chlorine atom
b) denotes an alkanoyl group

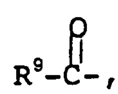

in which $R^9$ is a methyl or ethyl radical which can be substituted by one hydroxyl group or 1 to 3 fluorine atoms
c) denotes a carboxyl group, or the methyl or ethyl ester, or methyl- or dimethylamide, or sodium, potassium or ammonium salt thereof, or
d) denotes hydrogen

$R^8$ denotes the structural elements of the formulae VI or VII

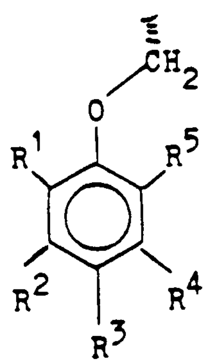

VI

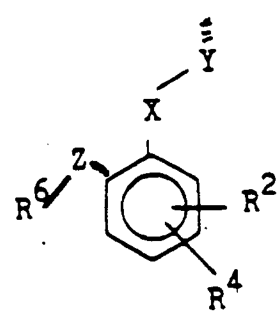

VII

in which

- X-Y represents a radical of the formula trans -CH=CH-, and
- Z represents a $CH_2$ group,
- $R^1$ and $R^5$ are identical or different and
  - a) represent hydrogen or chlorine, or
  - b) represent a straight-chain or branched alkyl radical with 1 to 6 carbon atoms, it being possible for the alkyl radical in turn to be mono- or disubstituted by phenyl or phenoxy radicals, which can in turn be substituted in the nucleus by fluorine or chlorine,
- $R^2$ and $R^4$ are identical or different and are hydrogen, methyl, ethyl, fluorine or chlorine,
- $R^3$ represents hydrogen, methyl or chlorine, and
- $R^6$ represents a cycloaliphatic hydrocarbon radical with 5 to 6 carbon atoms or a phenyl radical which can be substituted in the nucleus by fluorine, chlorine, trifluoromethyl, methyl, ethyl, methoxy or ethoxy.

**3.** A compound as claimed in claim 1, in which, in the formula V or V',

- A denotes -S-
- $R^7$
  - a) denotes an alkyl radical which has 1 or 2 carbon atoms and can be substituted by
    - aa) a hydroxyl group
    - ab) an amino or ammonium group
    - ac) a carboxyl group, or the methyl ester, or sodium, potassium or ammonium salt thereof
    - ad) a phenyl radical
    - ae) a fluorine atom, or
  - b) denotes an acetyl or trifluoroacetyl group
- $R^8$ denotes the structural elements of the formulae VI or VII

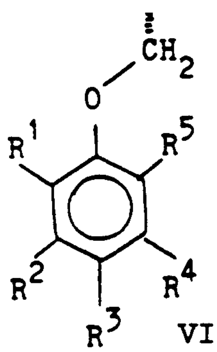
VI

or

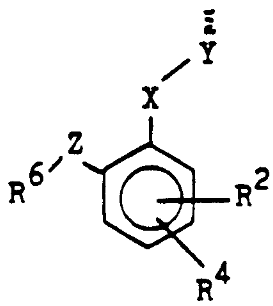
VII

in which

- X-Y is a radical of the formula trans -CH=CH- and
- Z is a $-CH_2-$ group,
- $R^1$ and $R^5$ are identical or different and
  - a) are chlorine, or
  - b) are a straight-chain or branched alkyl radical with 1 to 3 carbon atoms, it being possible for the alkyl radical in turn to be mono- or disubstituted by phenyl or phenoxy radicals, which can in turn be substituted in the nucleus by fluorine, and
- $R^2$ and $R^4$ are hydrogen
- $R^3$ is chlorine or methyl and
- $R^6$ is cyclohexyl, an unsubstituted phenyl radical or a phenyl radical which is substituted by chlorine or fluorine.

**4.** A process for the preparation of a compound as claimed in claim 1, which comprises

a) reacting a compound of the formula I or II

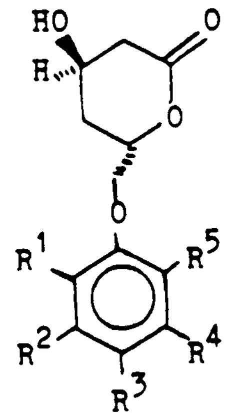

I

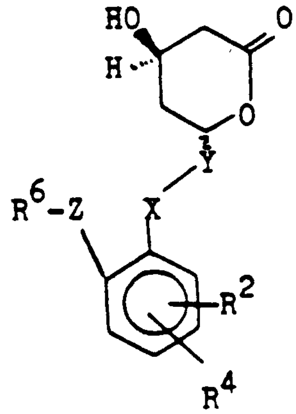

II

in which $R^1$, $R^2$, $R^3$ $R^4$, $R^5$, $R^6$, X, Y and Z have the meaning given in the case of formula V, with a dehydrating reagent to give an $\alpha,\beta$-unsaturated lactone of the formula III or IV

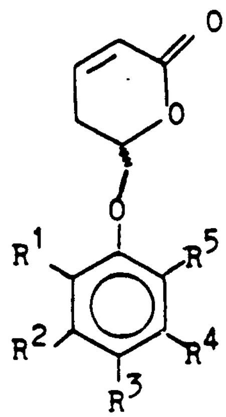

III

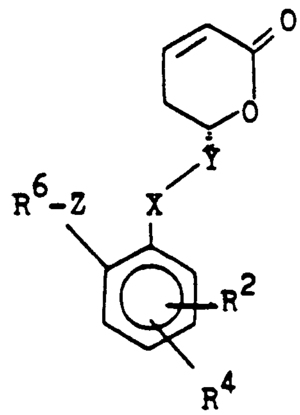

IV

or converting a precursor of the formula I' or II'

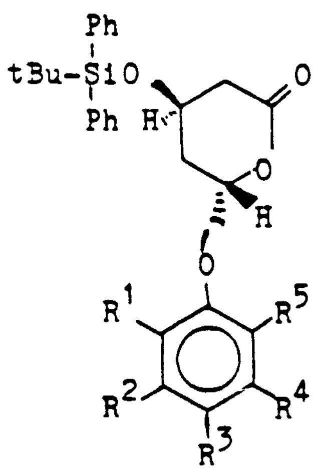

I'

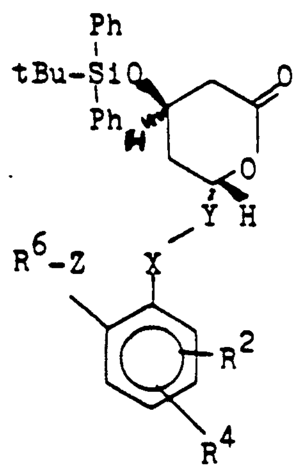

II'

in which $R^1$ to $R^6$, X, Y and Z have the meanings given in the case of formula V, into an $\alpha,\beta$-unsaturated lactone of the formula III or IV by desiloxylation, and

b) reacting the $\alpha,\beta$-unsaturated lactone of the formula III or IV with a nucleophilic compound $R^7AH$ to give a compound of the formula V in which $R^7$ and A have the meanings given in the case of formula V and $R^8$ denotes the structural element VI, in which $R^1$ to $R^5$ have the meanings given, or

the structural element VII, in which $R^2$, $R^4$ and $R^6$ have the meanings given and X-Y represents the trans CH = CH group, and
if appropriate, hydrogenating a resulting compound V, in which X-Y represents the trans CH = CH group, to give a compound V in which X-Y represents the $CH_2$-$CH_2$ group, if appropriate converting a resulting compound of the formula V into the open-chain hydroxycarboxylic acid of the formula V' or an ester or salt thereof, or if appropriate converting a salt or an ester into the free hydroxycarboxylic acid.

**5.** A pharmaceutical product containing a compound as claimed in claim 1.

**6.** The use of a compound as claimed in claim 1 for the preparation of medicaments for the prophylaxis and therapy of hypercholesterolemia.

**7.** An α,β-unsaturated lactone of the formula III

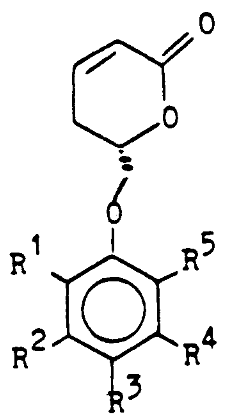

in which $R^1$ to $R^5$ have the meanings given in claim 1.

**8.** An α,β-unsaturated lactone of the formula IV

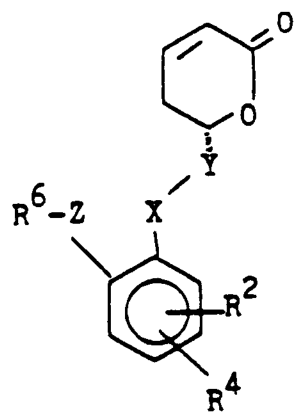

in which $R^2$, $R^4$, $R^6$, X, Y and Z have the meanings given in claim 1.

**Claims for the following Contracting States : AT, ES, GR**

**1.** A process for the preparation of a 4(R)-substituted 6(S)-phenyloxymethyl-, 6(S)-β-phenylethyl- and 6-(S)-β-styryl-tetrahydropyran-2-one of the formula V

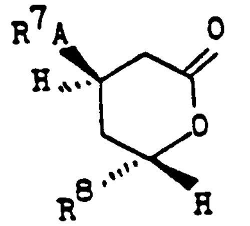

V

in which:

A denotes -S-, -NH-, $NR^7$ or $-CHR^7-$

$R^7$

a) denotes a straight-chain or branched alkyl radical which has 1 - 3 carbon atoms and can be substituted

aa) by a hydroxyl group

ab) by an amino or ammonium group

ac) by a carboxyl group $CO_2H$ or the methyl, ethyl or benzyl ester or methyl- or dimethylamide or sodium, potassium or ammonium salt thereof

ad) by a phenyl radical

ae) by 1 to 3 halogen atoms

b) denotes an alkanoyl group

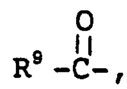

in which $R^9$ is a straight-chain or branched alkyl radical which has 1 to 4 carbon atoms and can be substituted by the groups mentioned under aa) to ae)

c) denotes a carboxyl group, the methyl, ethyl or benzyl ester, or methyl- or dimethylamide, or sodium, potassium or ammonium salt thereof, or

d) denotes hydrogen

$R^8$ denotes the structural elements of the formulae VI or VII

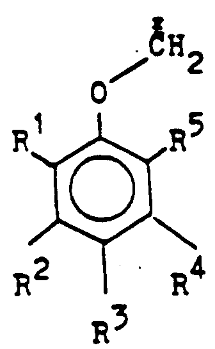

or

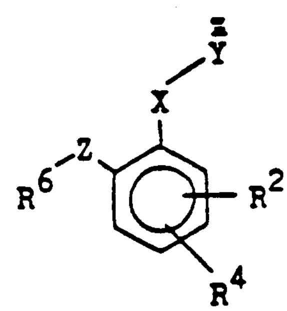

VI VII

in which

X-Y denotes a radical of the formula trans -CH=CH- or $-CH_2-CH_2-$

Z denotes a $-CH_2-$ or $-CH_2-CH_2-$ group

$R^1$ and $R^5$ are identical or different and

a) denote hydrogen or halogen

b) denote cycloalkyl with 4 to 8 carbon atoms or a phenyl radical, which can be mono-, di- or tri- substituted in the nucleus by halogen, trifluoromethyl and/or alkyl or alkoxy with in each case 1 to 4 carbon atoms, or

c) denote a straight-chain or branched alkyl radical with 1 to 18 carbon atoms or a straight-chain or branched alkenyl radical with 2 to 18 carbon atoms, it being possible for the alkyl and alkenyl radicals in turn to be mono-, di- or trisubstituted by

α) straight-chain or branched alkoxy radicals with up to 10 carbon atoms or cycloalkoxy radicals with 3 to 7 carbon atoms or straight-chain or branched alkenyloxy or alkynyloxy radicals with 3 to 6 carbon atoms,

β) halogen, hydroxyl, cycloalkyl with 3 to 7 carbon atoms, unsubstituted phenyl or α- or β-thienyl radicals, or phenyl or α- or β-thienyl radicals which are in turn mono-, di- or trisubstituted in the nucleus by halogen, trifluoromethyl and/or alkyl or alkoxy with 1 to 4 carbon atoms,

γ) unsubstituted phenoxy, benzyloxy or α- or β-thienyloxy radicals, or phenoxy, benzyloxy or α- or β-thienyloxy radicals which are in turn mono-, di- or trisubstituted in the nucleus by halogen, trifluoromethyl and/or alkyl or alkoxy with 1 to 4 carbon atoms,

δ) the group

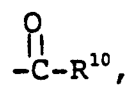

in which $R^{10}$ denotes: a straight-chain or branched alkyl or alkenyl radical with up to 8 carbon atoms, or a cycloalkyl or cycloalkenyl radical with in each case 3 to 8 carbon atoms, or an unsubstituted phenyl radical or a phenyl radical which is in turn mono-, di- or trisubstituted in the nucleus by halogen, trifluoromethyl and/or alkyl or alkoxy with 1 to 4 carbon atoms, or a pyridyl radical,

$R^2$ and $R^4$ are identical or different and denote hydrogen, alkyl with 1 to 4 carbon atoms, halogen or alkoxy with 1 to 4 carbon atoms, and

$R^3$ denotes hydrogen, alkyl or alkenyl with up to 4 carbon atoms, halogen or alkoxy with 1 to 4 carbon atoms, and

$R^6$ denotes a cycloaliphatic hydrocarbon with 3 to 7 carbon atoms, a phenyl radical which can be mono, di- or trisubstituted in the nucleus by halogen, trifluoromethyl, alkyl or alkoxy with in each case 1 to 6 carbon atoms or hydroxymethyl, or a furyl, thienyl or pyridyl radical, it being possible for the heteroaromatic radicals to be mono- or disubstituted by halogen, trifluoromethyl or alkyl or alkoxy with in each case 1 to 6 carbon atoms,

and the corresponding open-chain hydroxycarboxylic acid of the formula V'

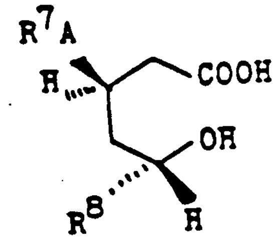

V'

in which A, $R^7$ and $R^8$ have the meanings given in the case of formula V, or a pharmacologically acceptable salt thereof with a base or a pharmacologically acceptable ester thereof, which comprises

a) reacting a compound of the formula I or II

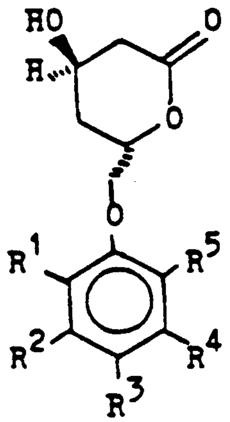

I

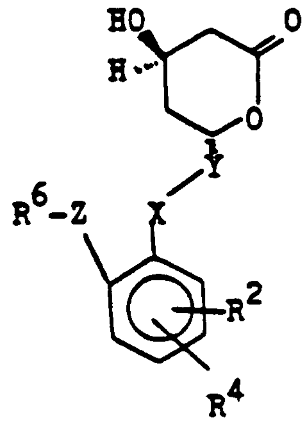

II

in which $R^1$, $R^2$, $R^3$, $R^4$, $R^5$, $R^6$, X, Y and Z have the meaning given in the case of formula V, with a dehydrating reagent to give an $\alpha,\beta$-unsaturated lactone of the formula III or IV

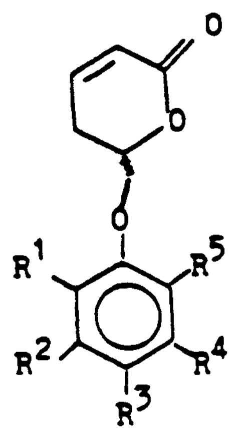

III

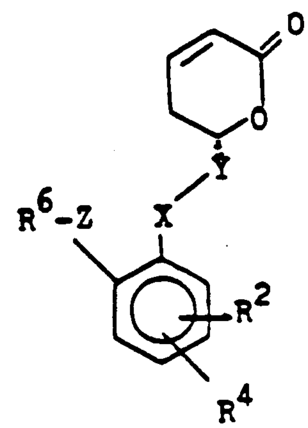

IV

or converting a precursor of the formula I' or II'

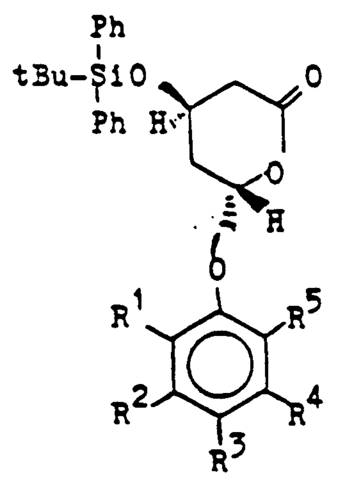

I'

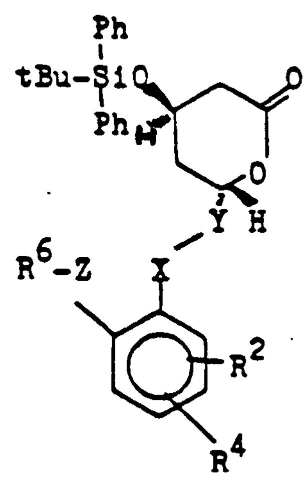

II'

in which $R^1$ to $R^6$, X, Y and Z have the meanings given in the case of formula V, into an $\alpha$, $\beta$-unsaturated lactone of the formula III or IV by desiloxylation, and
b) reacting the $\alpha,\beta$-unsaturated lactone of the formula III or IV with a nucleophilic compound $R^7AH$ to give a compound of the formula V in which $R^7$ and A have the meanings given in the case of formula V and $R^8$ denotes the structural element VI,

in which $R^1$ to $R^5$ have the meanings given, or the structural element VII, in which $R^2$, $R^4$ and $R^6$ have the meanings given and X-Y represents the trans CH = CH group, and
if appropriate, hydrogenating a resulting compound V, in which X-Y represents the trans CH = CH group, to give a compound V in which X-Y represents the $CH_2$-$CH_2$ group, if appropriate converting a resulting compound of the formula V into the open-chain hydroxycarboxylic acid of the formula V' or an ester or salt thereof, or if appropriate converting a salt or an ester into the free hydroxycarboxylic acid.

**2.** A process as claimed in claim 1, wherein a compound of the formula V or V', in which

A denotes -S-

$R^7$
- a) denotes a straight-chain or branched alkyl radical which has 1 or 2 carbon atoms and can be substituted by
  - aa) a hydroxyl group
  - ab) an amino or ammonium group
  - ac) a carboxyl group, or the methyl, ethyl or benzyl ester, or methyl- or dimethylamide, or sodium, potassium or ammonium salt thereof
  - ad) a phenyl radical
  - ae) a fluorine or chlorine atom
- b) denotes an alkanoyl group

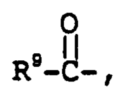,

  in which $R^9$ is a methyl or ethyl radical which can be substituted by one hydroxyl group or 1 to 3 fluorine atoms
- c) denotes a carboxyl group, or the methyl or ethyl ester, or methyl- or dimethylamide, or sodium, potassium or ammonium salt thereof, or
- d) denotes hydrogen

$R^8$ denotes the structural elements of the formulae VI or VII

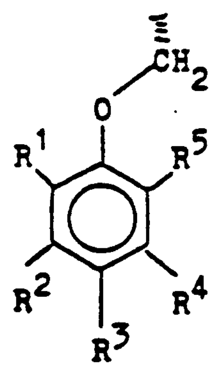 VI

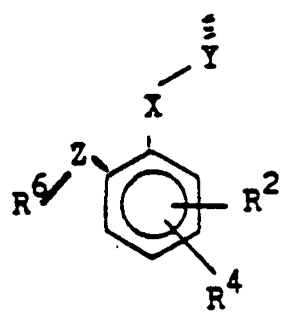 VII

in which

X-Y represents a radical of the formula trans -CH = CH-, and

Z represents a $CH_2$ group,

$R^1$ and $R^5$ are identical or different and
- a) represent hydrogen or chlorine, or
- b) represent a straight-chain or branched alkyl radical with 1 to 6 carbon atoms, it being possible for the alkyl radical in turn to be mono- or disubstituted by phenyl or phenoxy radicals, which can in turn be substituted in the nucleus by fluorine or chlorine,

$R^2$ and $R^4$ are identical or different and are hydrogen, methyl, ethyl, fluorine or chlorine,

$R^3$ represents hydrogen, methyl or chlorine, and

$R^6$ represents a cycloaliphatic hydrocarbon with 5 to 6 carbon atoms or a phenyl radical which can be substituted in the nucleus by fluorine, chlorine, trifluoromethyl, methyl, ethyl, methoxy or ethoxy, or an ester or salt thereof is prepared.

**3.** A process as claimed in claim 1, wherein a compound of the formula V or V', in which

A denotes -S-

$R^7$

a) denotes an alkyl radical which has 1 or 2 carbon atoms and can be substituted by

aa) a hydroxyl group

ab) an amino or ammonium group

ac) a carboxyl group, or the methyl ester, or sodium, potassium or ammonium salt thereof

ad) a phenyl radical

ae) a fluorine atom, or

b) denotes an acetyl or trifluoroacetyl group

$R^8$ denotes the structural elements of the formulae VI or VII

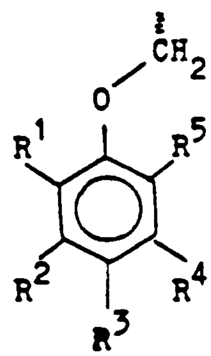

VI

or

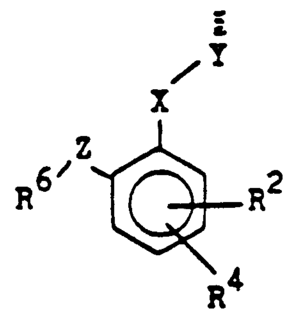

VII

in which

X-Y is a radical of the formula trans -CH = CH- and

Z is a $-CH_2-$ group,

$R^1$ and $R^5$ are identical or different and

a) are chlorine, or

b) are a straight-chain or branched alkyl radical with 1 to 3 carbon atoms, it being possible for the alkyl radical in turn to be mono- or disubstituted by phenyl or phenoxy radicals, which can in turn be substituted in the nucleus by fluorine, and

$R^2$ and $R^4$ are hydrogen

$R^3$ is chlorine or methyl and

$R^6$ is cyclohexyl, an unsubstituted phenyl radical or a phenyl radical which is substituted by chlorine or fluorine, or an ester or salt thereof is prepared.

**4.** A process for the preparation of a pharmaceutical product, which comprises converting a compound obtainable by the process as claimed in claim 1 into a suitable administration form.

**5.** A process as claimed in claim 1, wherein the precursor of the formula III

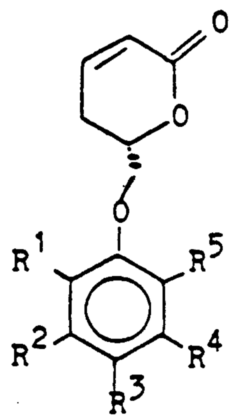

III

in which $R^1$ to $R^5$ have the meanings given in claim 1 in the case of formula V, is isolated.

**6.** A process as claimed in claim 1, wherein the precursor of the formula IV

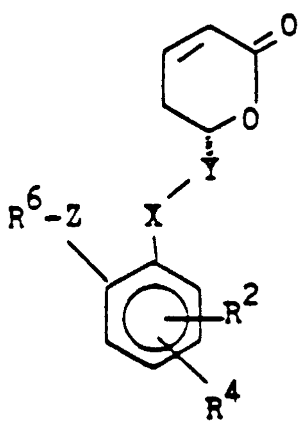

IV

in which $R^2$, $R^4$, $R^6$, X, Y and Z have the meanings given in the claim 1 in the case of formula V, is isolated.

**Revendications**

**Revendications pour les Etats contractants suivants : BE, CH, DE, FR, GB, IT, LI, LU, NL, SE**

**1.** 6(S)-phényloxyméthyl-6(S)-$\beta$-phényléthyl- et 6(S)-$\beta$-styryl-tétrahydropyranne-2-ones 4(R)-substituées de formule générale V

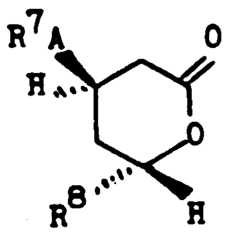

V

dans laquelle

A représente -S-, -NH-, $NR^7$ ou $-CHR^7-$,

$R^7$ représente

a) un radical alkyle à chaîne droite ou ramifiée ayant de 1 à 3 atomes de carbone,

qui peut être substitué

aa) par un groupe hydroxy,

ab) par un groupe amino ou ammonium,

ac) par un groupe carboxy $CO_2H$, son ester méthylique, éthylique ou benzylique, son méthyl- ou diméthylamide, son sel sodique, potassique ou d'ammonium,

ad) par un groupe phényle,

ae) par de 1 à 3 atomes d'halogène,

b) un groupe alcanoyle

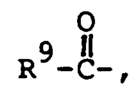

dans lequel $R^9$ est un radical alkyle à chaîne droite ou ramifiée ayant de 1 à 4 atomes de carbone, qui peut être substitué par les groupes indiqués en aa) à ae),

c) un groupe carboxy, son ester méthylique, éthylique ou benzylique, son méthyl- ou diméthylamide, son sel sodique, potassique ou d'ammonium,

d) un atome d'hydrogène,

$R^8$ représente les éléments structuraux de formules VI ou VII

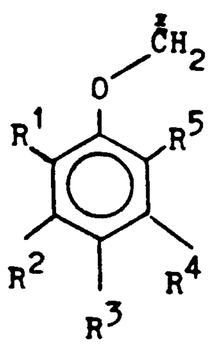

VI

ou

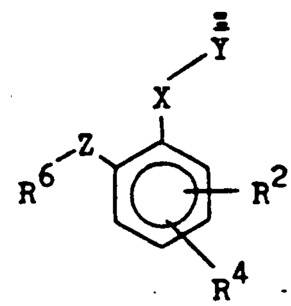

VII

formules dans lesquelles

X-Y représente un radical de formule trans -CH = CH- ou $-CH_2-CH_2$,

Z représente un groupe $-CH_2-$ ou $-CH_2-CH_2-$,

$R^1$, $R^5$ sont identiques ou différents et représentent

a) un atome d'hydrogène ou d'halogène,

b) un radical cycloalkyle ayant de 4 à 8 atomes de carbone ou un radical phényle, qui peut être une à trois fois substitué au noyau par un ou des atomes d'halogène ou groupes trifluorométhyle et/ou alkyle ou alcoxy ayant chacun de 1 à 4 atomes de carbone, ou

c) un radical alkyle à chaîne droite ou ramifiée ayant de 1 à 18 atomes de carbone ou un radical alcényle à chaîne droite ou ramifiée ayant de 2 à 18 atomes de carbone, les radicaux alkyle ou alcényle pouvant pour leur part être 1 à 3 fois substitués par

α) un ou des groupes alcoxy à chaînes droites ou ramifiées ayant jusqu'à 10 atomes de carbone, ou groupes cycloalcoxy ayant de 3 à 7 atomes de carbone, ou groupes alcényloxy ou alcynyloxy ayant de 3 à 6 atomes de carbone,

β) un ou des atomes d'halogène ou groupes hydroxy, cycloalkyle ayant de 3 à 7 atomes de carbone, groupes phényle, α- ou β-thiényle non substitués ou groupes phényle, α- ou β-thiényle qui sont 1 à 3 fois substitués au noyau par un ou des atomes d'halogène ou groupes trifluorométhyle et/ou alkyle ou alcoxy ayant de 1 à 4 atomes de carbone,

γ) un ou des groupes phénoxy, benzyloxy, α- ou β-thiényloxy non substitués, ou groupes phénoxy, benzyloxy, α- ou β-thiényloxy qui pour leur part sont 1 à 3 fois

substitués au noyau par un ou des atomes d'halogène ou groupes trifluorométhyle et/ou alkyle ou alcoxy ayant de 1 à 4 atomes de carbone,

δ) le groupe

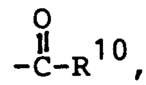

$R^{10}$ représentant:
un radical alkyle ou alcényle à chaîne droite ou ramifiée ayant jusqu'à 8 atomes de carbone, ou un radical cycloalkyle ou cycloalcényle ayant chacun de 3 à 8 atomes de carbone, ou un radical phényle non substitué ou un radical phényle qui pour sa part est 1 à 3 fois substitué au noyau par un ou des atomes d'halogène ou groupes trifluorométhyle, et/ou alkyle ou alcoxy ayant de 1 à 4 atomes de carbone, ou un radical pyridyle,

$R^2$ et $R^4$ sont identiques ou différents, et représentent un atome d'hydrogène ou d'halogène ou un groupe alkyle ayant de 1 à 4 atomes de carbone ou alcoxy ayant de 1 à 4 atomes de carbone, et

$R^3$ représente un atome d'hydrogène ou d'halogène, ou un groupe alkyle ou alcényle ayant jusqu'à 4 atomes de carbone ou alcoxy ayant de 1 à 4 atomes de carbone,

$R^6$ représente un radical hydrocarboné cycloaliphatique ayant de 3 à 7 atomes de carbone, un radical phényle qui peut être 1 à 3 fois substitué au noyau par un ou des atomes d'halogène ou groupes trifluorométhyle, alkyle ou alcoxy ayant chacun de 1 à 6 atomes de carbone ou par hydroxyméthyle, ou un radical furyle, thiényle ou pyridyle, les radicaux hétéroaromatiques pouvant être 1 ou 2 fois substitués par un ou des atomes d'halogène ou groupes trifluorométhyle, alkyle ou alcoxy ayant chacun de 1 à 6 atomes de carbone,

ainsi que les acides hydroxycarboxyliques à chaîne ouverte correspondants, de formule V'

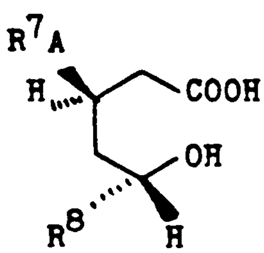

V'

dans laquelle A, $R^7$ et $R^8$ on les significations données à propos de la formule V,
leurs sels pharmacologiquement acceptables avec des bases et leurs esters pharmacologiquement acceptables.

**2.** Composés selon la revendication 1, caractérisés en ce que, dans la formule générale V ou V',

A représente -S-,

$R^7$ représente

a) un radical alkyle à chaîne droite ou ramifiée ayant 1 ou 2 atomes de carbone, qui peut être substitué par

aa) un groupe hydroxy,

ab) un groupe amino ou ammonium,

ac) un groupe carboxy, son ester méthylique, éthylique ou benzylique, son méthyl- ou diméthylamide, son sel sodique, potassique ou d'ammonium,

ad) un groupe phényle,

ae) un atome de fluor ou de chlore,

b) un groupe alcanoyle

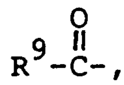

dans lequel $R^9$ est un radical méthyle ou éthyle qui peut être substitué par un groupe hydroxy ou 1 à 3 atomes de fluor,

c) un groupe carboxy, son ester méthylique ou éthylique, son méthyl- ou diméthylamide, son sel sodique, potassique ou d'ammonium,

d) un atome d'hydrogène,

$R^8$ représente les éléments structuraux de formule VI ou VII

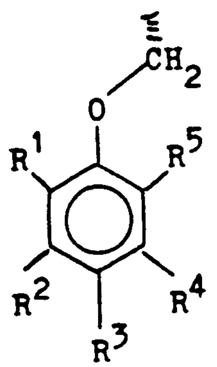

VI

ou

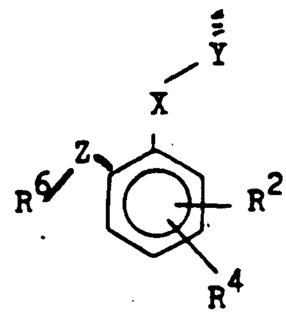

VII

formules dans lesquelles

X-Y représente un radical de formule trans -CH = CH- et

Z représente le groupe $CH_2$,

$R^1$, $R^5$ sont identiques ou différents, et représentent

a) un atome d'hydrogène ou de chlore,

b)un radical alkyle à chaîne droite ou ramifiée ayant de 1 à 6 atomes de carbone, le radical alkyle pouvant pour sa part être 1 ou 2 fois substitué par un ou des groupes phényle ou phénoxy, qui peuvent pour leur part être substitués au noyau par des atomes de fluor ou de chlore,

$R^2$, $R^4$ sont identiques ou différents et représentent un atome d'hydrogène, de fluor ou de chlore ou le groupe méthyle ou éthyle,

$R^3$ représente un atome d'hydrogène ou de chlore ou le groupe méthyle,

$R^6$ représente un radical hydrocarboné cycloaliphatique ayant 5 ou 6 atomes de carbone, un radical phényle qui peut être substitué au noyau par un atome de fluor ou de chlore ou par le groupe trifluorométhyle, méthyle, éthyle, méthoxy, éthoxy.

**3.** Composés selon la revendication 1, caractérisés en ce que, dans la formule générale V ou V',

A représente -S-,

$R^7$ représente

a) un radical alkyle ayant 1 ou 2 atomes de carbone, qui peut être substitué par

aa) un groupe hydroxy,

ab) un groupe amino ou ammonium,

ac) un groupe carboxy, son ester méthylique, son sel sodique, potassique ou d'ammonium,

ad) un radical phényle,

ae) un atome de fluor,

b) un groupe acétyle ou trifluoroacétyle,

$R^8$ représente les éléments structuraux de formule VI ou VII

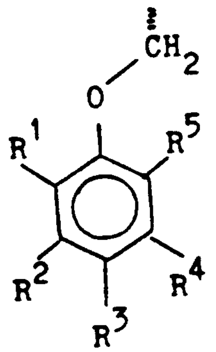

VI

ou

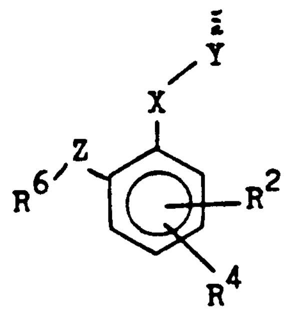

VII

formules dans lesquelles

X-Y représente un radical de formule trans -CH = CH- et

Z représente un groupe $-CH_2-$,

$R^1$, $R^5$ sont identiques ou différents, et représentent

a) un atome de chlore,

b)un radical alkyle à chaîne droite ou ramifiée ayant de 1 à 3 atomes de carbone, le radical alkyle pouvant pour sa part être 1 à 2 fois substitué par un ou des groupes phényle ou phénoxy qui peuvent pour leur part être substitué au noyau par un atome de fluor, et

$R^2$, $R^4$ sont des atomes d'hydrogène,

$R^3$ est un atome de chlore ou le groupe méthyle, et

$R^6$ est un radical cyclohexyle, phényle non substitué ou un radical phényle substitué par un atome de chlore ou de fluor.

**4.** Procédé pour la préparation des composés selon la revendication 1, caractérisé en ce que

a) on fait réagir des composés de formules générales I ou II

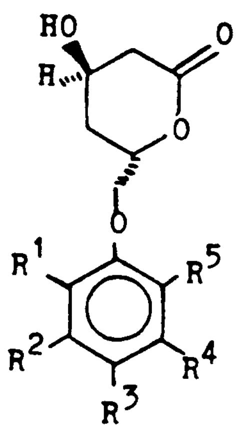

I

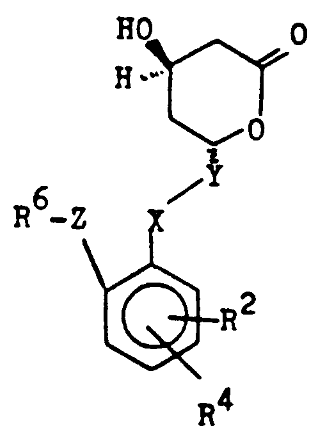

II

dans lesquelles $R^1$, $R^2$, $R^3$, $R^4$, $R^5$, $R^6$, X, Y, Z ont les significations données à propos de la formule V, avec un agent de déshydratation, pour aboutir aux lactones $\alpha,\beta$-insaturées de formules générales III ou IV

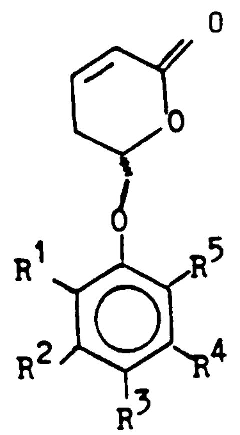

III

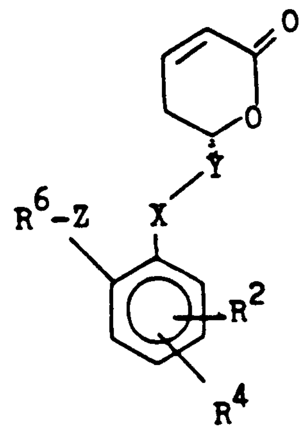

IV

ou on convertit les précurseurs, de formules I' et II'

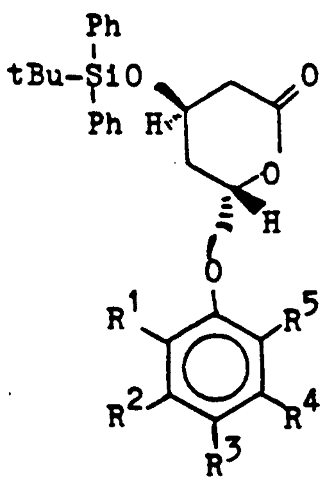

I'

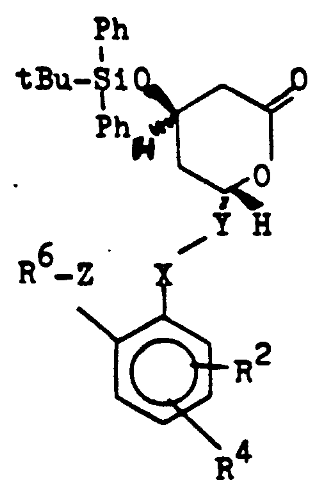

II'

dans lesquelles $R^1$ à $R^6$, X, Y et Z ont les significations données à propos de la formule V, par désiloxylation, en les lactones $\alpha,\beta$-insaturées de formules III ou IV, et

b) on fait réagir les lactones $\alpha,\beta$-insaturées de formules III ou IV avec des composés nucléophiles $R^7AH$, pour aboutir aux composés de formule V dans lesquels $R^7$ et A ont les significations données à propos de la fomule V et $R^8$ représente l'élément structural VI dans lequel $R^1$ à $R^5$ ont les significations indiquées, ou l'élément structural VII dans lequel $R^2$, $R^4$ et $R^6$ ont les significations indiquées et X-Y représente le groupe trans CH = CH, et

éventuellement on soumet à une hydrogénation un composé V obtenu, dans lequel X-Y représente le groupe trans CH = CH, pour aboutir à un composé V dans lequel X-Y représente le groupe $CH_2$-$CH_2$, éventuellement on convertit un composé de formule V obtenu en l'acide hydroxycarboxylique à chaîne ouverte, de formule V', ou en un de ses sels ou esters, ou éventuellement on convertit un sel ou un ester en l'acide hydroxycarboxylique libre.

**5.** Compositions pharmaceutiques contenant un composé selon la revendication 1.

**6.** Utilisation d'un composé selon la revendication 1, pour la fabrication de médicaments pour la prophylaxie et le traitement de l'hypercholestérolémie.

**7.** Lactones $\alpha,\beta$-insaturées de formule III

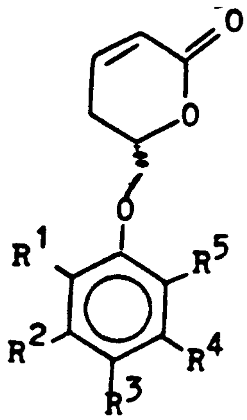

III

dans laquelle $R^1$ à $R^5$ ont les significations données dans la revendication 1.

**8.** Lactones $\alpha,\beta$-insaturées de formule IV

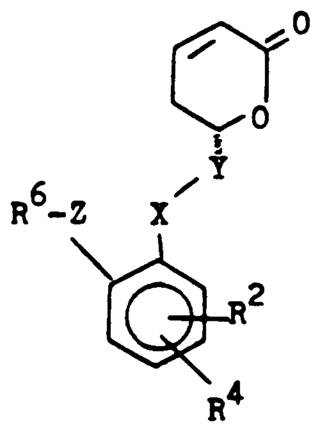

IV

dans laquelle $R^2$, $R^4$, $R^6$, X, Y et Z ont les significations données dans la revendication 1.

**Revendications pour les Etats contractants suivants : AT, ES, GR**

**1.** Procédé pour la préparation de 6(S)-phényloxyméthyl-6(S)-$\beta$-phényléthyl- et 6(S)-$\beta$-styryl-tétrahydropyranne-2-ones 4(R)-substituées de formule générale V

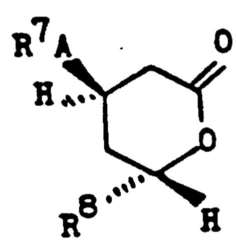

V

dans laquelle

A représente -S-, -NH-, $NR^7$ ou $-CHR^7-$,

$R^7$ représente

a) un radical alkyle à chaîne droite ou ramifiée ayant de 1 à 3 atomes de carbone, qui peut être substitué

aa) par un groupe hydroxy,

ab) par un groupe amino ou ammonium,

ac) par un groupe carboxy $CO_2H$, son ester méthylique, éthylique ou benzylique, son méthyl- ou diméthylamide, son sel sodique, potassique ou d'ammonium,

ad) par un groupe phényle,

ae) par de 1 à 3 atomes d'halogène,

b) un groupe alcanoyle

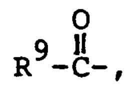

dans lequel $R^9$ est un radical alkyle à chaîne droite ou ramifiée ayant de 1 à 4 atomes de carbone, qui peut être substitué par les groupes indiqués en aa) à ae),

c) un groupe carboxy, son ester méthylique, éthylique ou benzylique, son méthyl- ou diméthylamide, son sel sodique, potassique ou d'ammonium,

d) un atome d'hydrogène,

$R^8$ représente les éléments structuraux de formules VI ou VII

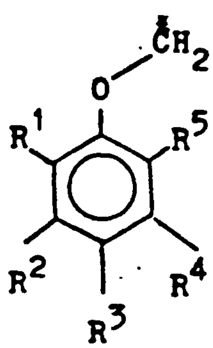

VI

ou

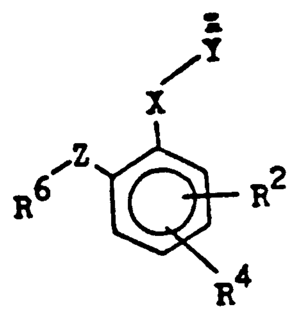

VII

dans lesquelles

X-Y représente un radical de formule trans $-CH=CH-$ ou $-CH_2-CH_2$,

Z représente un groupe $-CH_2-$ ou $-CH_2-CH_2-$,

$R^1$, $R^5$ sont identiques ou différents et représentent

a) un atome d'hydrogène ou d'halogène,

b) un radical cycloalkyle ayant de 4 à 8 atomes de carbone ou un radical phényle, qui peut être une à trois fois substitué au noyau par un ou des atomes d'halogène ou groupes trifluorométhyle et/ou alkyle ou alcoxy ayant chacun de 1 à 4 atomes de carbone, ou

c) un radical alkyle à chaîne droite ou ramifiée ayant de 1 à 18 atomes de carbone ou un radical alcényle à chaîne droite ou ramifiée ayant de 2 à 18 atomes de carbone, les radicaux alkyle ou alcényle pouvant pour leur part être 1 à 3 fois substitués par

α) un ou des groupes alcoxy à chaînes droites ou ramifiées ayant jusqu'à 10 atomes de carbone, ou groupes cycloalcoxy ayant de 3 à 7 atomes de carbone, ou groupes alcényloxy ou alcynyloxy ayant de 3 à 6 atomes de carbone,

β) un ou des atomes d'halogène ou groupes hydroxy, cycloalkyle ayant de 3 à 7 atomes de carbone, groupes phényle, α- ou β-thiényle non substitués ou groupes phényle, α- ou β-thiényle qui sont 1 à 3 fois substitués au noyau par un ou des atomes d'halogène ou groupes trifluorométhyle et/ou alkyle ou alcoxy ayant de 1 à 4 atomes de carbone,

γ) un ou des groupes phénoxy, benzyloxy, α- ou β-thiényloxy non substitués, ou groupes phénoxy, benzyloxy, α- ou β-thiényloxy qui pour leur part sont 1 à 3 fois substitués au noyau par un ou des atomes d'halogène ou groupes trifluorométhyle et/ou alkyle ou alcoxy ayant de 1 à 4 atomes de carbone,

δ) le groupe

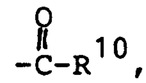

$R^{10}$ représentant:

un radical alkyle ou alcényle à chaîne droite ou ramifiée ayant jusqu'à 8 atomes de carbone, ou un radical cycloalkyle ou cycloalcényle ayant chacun de 3 à 8 atomes de carbone, ou un radical phényle non substitué ou un radical phényle qui pour sa part est 1 à 3 fois substitué au noyau par un ou des atomes d'halogène ou groupes trifluorométhyle, et/ou alkyle ou alcoxy ayant de 1 à 4 atomes de carbone, ou un radical pyridyle,

$R^2$ et $R^4$ sont identiques ou différents, et représentent un atome d'hydrogène ou d'halogène ou un groupe alkyle ayant de 1 à 4 atomes de carbone ou alcoxy ayant de 1 à 4 atomes de carbone, et

$R^3$ représente un atome d'hydrogène ou d'halogène, ou un groupe alkyle ou alcényle ayant jusqu'à 4 atomes de carbone ou alcoxy ayant de 1 à 4 atomes de carbone,

$R^6$ représente un radical hydrocarboné cycloaliphatique ayant de 3 à 7 atomes de carbone, un radical phényle qui peut être 1 à 3 fois substitué au noyau par un ou des atomes d'halogène ou groupes trifluorométhyle, alkyle ou alcoxy ayant chacun de 1 à 6 atomes de carbone, ou par le groupe hydroxyméthyle, ou un radical furyle, thiényle ou pyridyle, les radicaux hétéroaromatiques pouvant être 1 ou 2 fois substitués par un ou des atomes d'halogène ou groupes trifluorométhyle, alkyle ou alcoxy ayant chacun de 1 à 6 atomes de car carbone,

ainsi que des acides hydroxycarboxyliques à chaîne ouverte correspondants, de formule V'

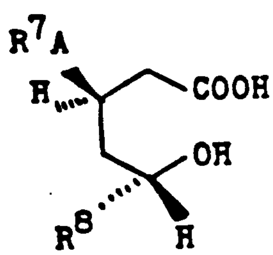

V'

dans laquelle A, $R^7$ et $R^8$ on les significations données à propos de la formule V,

de leurs sels pharmacologiquement acceptables avec des bases et de leurs esters pharmacologiquement acceptables,

caractérisé en ce que

a) on fait réagir des composés de formules générales I ou II

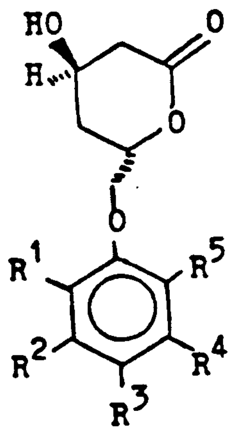

I

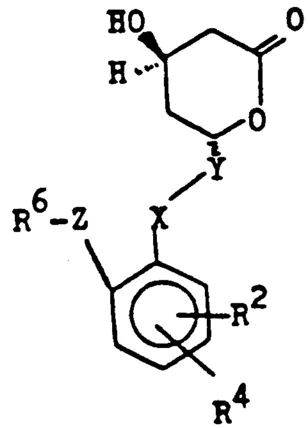

II

dans lesquelles $R^1$, $R^2$, $R^3$, $R^4$, $R^5$, $R^6$, X, Y, Z ont les significations données à propos de la formule V, avec un agent de déshydratation, pour aboutir aux lactones $\alpha,\beta$-insaturées de formules générales III ou IV

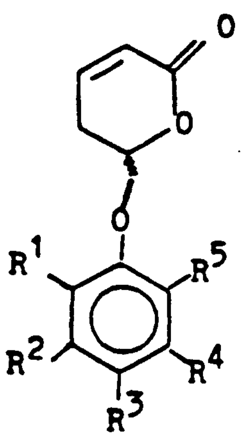

III

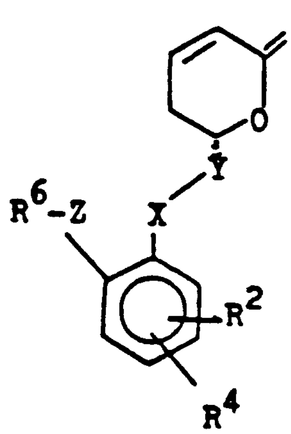

IV

ou on convertit les précurseurs, de formules I' et II'

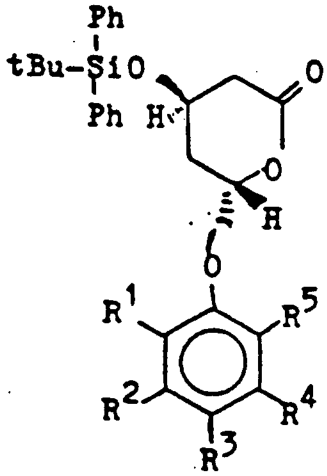

I'

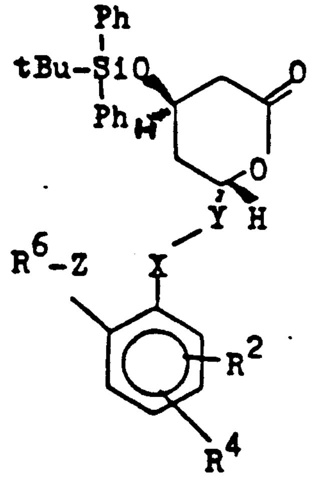

II'

dans lesquelles $R^1$ à $R^6$, X, Y et Z ont les significations données à propos de la formule V, par désiloxylation, en les lactones $\alpha,\beta$-insaturées de formules III ou IV, et

b) on fait réagir les lactones $\alpha,\beta$-insaturées de formules III ou IV avec des composés nucléophiles

$R^7AH$; pour aboutir aux composés de formule V dans lesquels $R^7$ et A ont les significations données à propos de la formule V et $R^8$ représente l'élément structural VI dans lequel $R^1$ à $R^5$ ont les significations indiquées, ou l'élément structural VII dans lequel $R^2$, $R^4$ et $R^6$ ont les significations indiquées et X-Y représente le groupe trans CH = CH, et
éventuellement on soumet à une hydrogénation un composé V obtenu, dans lequel X-Y représente le groupe trans CH = CH, pour aboutir à un composé V dans lequel X-Y représente le groupe $CH_2$-$CH_2$, éventuellement on convertit un composé de formule V obtenu en l'acide hydroxycarboxylique à chaîne ouverte, de formule V, ou en un de ses sels ou esters, ou éventuellement on convertit un sel ou un ester en l'acide hydroxycarboxylique libre.

**2.** Procédé selon la revendication 1, caractérisé en ce que l'on prépare un composé de formule générale V ou V' dans lequel

A représente -S-,

$R^7$ représente

a) un radical alkyle à chaîne droite ou ramifiée ayant 1 ou 2 atomes de carbone, qui peut être substitué par

aa) un groupe hydroxy,

ab) un groupe amino ou ammonium,

ac) un groupe carboxy, son ester méthylique, éthylique ou benzylique, son méthyl- ou diméthylamide, son sel sodique, potassique ou d'ammonium,

ad) un groupe phényle,

ae) un atome de fluor ou de chlore,

b) un groupe alcanoyle

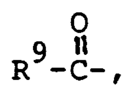

dans lequel $R^9$ est un radical méthyle ou éthyle qui peut être substitué par un groupe hydroxy ou 1 à 3 atomes de fluor,

c) un groupe carboxy, son ester méthylique ou éthylique, son méthyl- ou diméthylamide, son sel sodique, potassique ou d'ammonium,

d) un atome d'hydrogène,

$R^8$ représente les éléments structuraux de formule VI ou VII

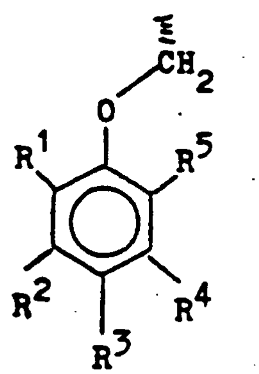

VI

ou

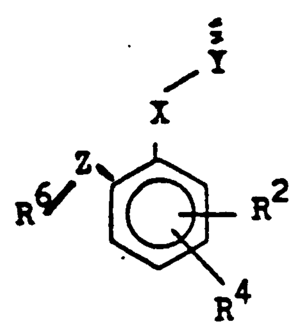

VII

formules dans lesquelles

X-Y représente un radical de formule trans -CH = CH- et

Z représente le groupe $CH_2$,

$R^1$, $R^5$ sont identiques ou différents, et représentent

a) un atome d'hydrogène ou de chlore,

b)un radical alkyle à chaîne droite ou ramifiée ayant de 1 à 6 atomes de carbone, le radical alkyle pouvant pour sa part être 1 ou 2 fois substitué par un ou des groupes phényle ou phénoxy, qui peuvent pour leur part être substitués au noyau par des

atomes de fluor ou de chlore,

$R^2$, $R^4$ sont identiques ou différents et représentent un atome d'hydrogène, de fluor ou de chlore ou le groupe méthyle ou éthyle,

$R^3$ représente un atome d'hydrogène ou de chlore ou le groupe méthyle,

$R^6$ représente un radical hydrocarboné cycloaliphatique ayant 5 ou 6 atomes de carbone, un radical phényle qui peut être substitué au noyau par un atome de fluor ou de chlore ou par le groupe trifluorométhyle, méthyle, éthyle, méthoxy, éthoxy,

ou ses sels ou esters.

**3.** Procédé selon la revendication 1, caractérisé en ce que l'on prépare un composé de formule générale V ou V', dans lequel

A représente -S-,

$R^7$ représente

a) un radical alkyle ayant 1 ou 2 atomes de carbone, qui peut être substitué par

aa) un groupe hydroxy,

ab) un groupe amino ou ammonium,

ac) un groupe carboxy, son ester méthylique, son sel sodique, potassique ou d'ammonium,

ad) un radical phényle,

ae) un atome de fluor,

b) un groupe acétyle ou trifluoroacétyle,

$R^8$ représente les éléments structuraux de formule VI ou VII

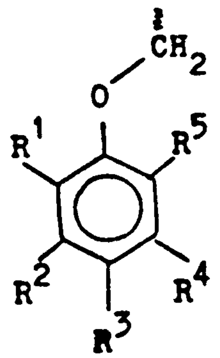

VI

ou

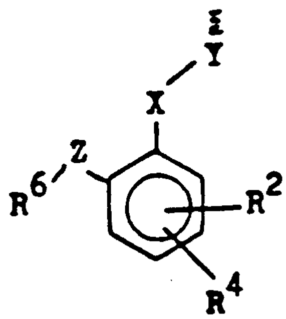

VII

formules dans lesquelles

X-Y représente un radical de formule trans -CH = CH- et

Z représente un groupe $-CH_2-$,

$R^1$, $R^5$ sont identiques ou différents, et représentent

a) un atome de chlore,

b)un radical alkyle à chaîne droite ou ramifiée ayant de 1 à 3 atomes de carbone, le radical alkyle pouvant pour sa part être 1 à 2 fois substitué par un ou des groupes phényle ou phénoxy qui peuvent pour leur part être substitués au noyau par un atome de fluor, et

$R^2$, $R^4$ sont des atomes d'hydrogène,

$R^3$ est un atome de chlore ou le groupe méthyle, et

$R^6$ est un radical cyclohexyle, phényle non substitué ou un radical phényle substitué par un atome de chlore ou de fluor,

ou ses sels ou esters.

**4.** Procédé pour la préparation d'une composition pharmaceutique, caractérisé en ce que l'on met sous une forme pharmaceutique appropriée un composé pouvant être obtenu conformément au procédé selon la revendication 1.

**5.** Procédé selon la revendication 1, caractérisé en ce que l'on isole les produits intermédiaires de formule

III

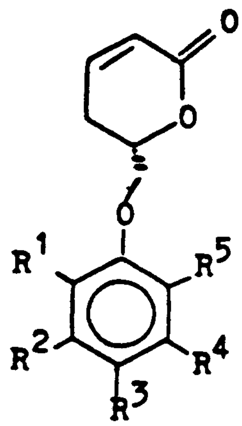

III

dans laquelle $R^1$ à $R^5$ ont les significations données à propos de la formule V dans la revendication 1.

**6.** Procédé selon la revendication 1, caractérisé en ce que l'on isole les produits intermédiaires de formule IV

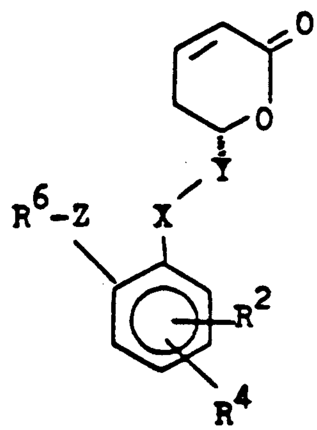

IV

dans laquelle $R^2$, $R^4$, $R^6$, X, Y et Z ont les significations données à propos de la formule V dans la revendication 1.